# EUROPÄISCHE PATENTANMELDUNG

(11) **EP 1 586 585 A1**
(43) Veröffentlichungstag der Anmeldung: **19.10.2005**
(21) Anmeldenummer: 04008881.7
(22) Anmeldetag: 14.04.2004
(51) Int. Cl.: C07K 14/54, C12N 15/11

(54) **Expressionssystem zur Herstellung von IL-15/Fc-Fusionsproteinen und ihre Verwendung**

(71) Anmelder: F. HOFFMANN-LA ROCHE AG, 4070 Basel (CH)
(72) Erfinder: Die Erfindernennung liegt noch nicht vor
(74) Vertreter: Bösl, Raphael Konrad

(57) **Zusammenfassung**

Die Erfindung betrifft ein Expressionssystem enthaltend eine oder mehrere Nukleinsäure(n) umfassend mindestens eine Nukleinsäure für ein Interleukin 15/Fc- (IL-15/Fc) -Fusionsprotein, mindestens einen Promotor, mindestens eine Nukleinsäure für einen CD5-Leader und ggf. mindestens eine Nukleinsäure für ein selektionierbares Markergen; eine Nukleinsäure umfassend die Bestandteile des genannten Expressionssystems sowie eine Wirtszelle enthaltend das Expressionssystem oder die Nukleinsäure. Weiterhin betrifft die Erfindung ein Verfahren zur Herstellung eines IL-15/Fc-Fusionsproteins unter Verwendung des Expressionssystems sowie die Verwendung des Expressionssystems, der Nukleinsäure, der Wirtszelle oder des CD5-Leaders zur Expression in Wirtszellen.

## Beschreibung

Die Erfindung betrifft ein Expressionssystem, das mindestens eine Nukleinsäure für ein Interleukin-15/Fc- (IL-15/Fc) -Fusionsprotein umfasst und mit dessen Hilfe das IL-15/Fc-Fusionsprotein hergestellt werden kann. Weiterhin betrifft die Erfindung ein Verfahren zur Herstellung eines IL-15/Fc-Fusionsproteins unter Verwendung des Expressionssystems sowie die Verwendung des Expressionssystems, der Nukleinsäure, der Wirtszelle oder des CD5-Leaders zur Expression von Proteinen in Wirtszellen.

In Säugetieren beruht das Immungeschehen auf einer Vielzahl komplexer zellulärer und azellulärer Interaktionen, die im Sinne eines Immun-Netzwerks funktionieren. Viele Mechanismen innerhalb dieses komplexen Netzwerks sind erst in jüngerer Zeit in ihrer Funktion aufgeklärt worden. Eine Schlüsselrolle als lösliche Botenstoffe innerhalb des Immun-Netzwerks spielen Cytokine, zu denen der 1994 beschriebene Faktor Interleukin-15 (Grabstein *et al*., 1994, Science 264: 965-968) gehört. Interleukin-15 (IL-15) beeinflusst als Immunmodulator, Wachstumsfaktor, Chemokin und Überlebensfaktor die Proliferation, Differenzierung, Aktivierung und das Überleben von Zellen des Immunsystems wie T-Zellen, Monocyten/Macrophagen, NK-Zellen und weiteren IL-15-sensitiven Zellen des Gewebes, wie Keratinocyten und anderen. Neben seiner Funktion als Immunmodulator spielt IL-15 auch eine Rolle bei der Regulation des Metabolismus von Muskel- und Fettgewebe.

Typischerweise bindet IL-15 an seine Effektorzellen über den heterotrimeren Interleukin-15-Rezeptor (IL-15R). Der IL-15R besteht aus einer spezifisch an IL-15 bindenden α-Untereinheit, einer ebenfalls durch IL-2 erkannten β-Untereinheit und einer γ-Untereinheit, die ebenfalls von weiteren Mitgliedern der Interleukin-Familie wie IL-2, IL-4, IL-7, IL-9 und IL-15 erkannt wird.

IL-15 spielt eine Rolle bei einer Vielzahl von Autoimmunerkrankungen und chronisch entzündlichen Erkrankungen, wie z.B. Rheumatischer Arthritis, Psoriasis, Multipler Sklerose, Morbus Crohn, Colitis ulcerosa, Enterocolitis, pulmonärer Sarcoidose oder systemischem Lupus Erythematodes, sowie bei der immunologischen Abstoßung transplantierter Organe, Gewebe und Zellen. Ebenso spielt IL-15 eine Rolle bei lymphoiden Leukämien.

Ein therapeutischer Einsatz von Interleukin-15 erfolgt entweder nach agonistischem Prinzip zur Expansion von Lymphocytenpopulationen bei Krebspatienten und bei Immundefizienz-Erkrankungen oder bei Erkrankungen mit einer pathologischen Aktivierung des Immunsystems nach antagonistischem Prinzip durch Einsatz von Agenzien, die die Wirkung von IL-15 blockieren. Dabei kann es sich um lösliche IL-15-Rezeptor-Polypeptide, gegen IL-15 oder den IL-15-Rezeptor gerichtete Antikörper oder um Fusionsproteine mit einem IL-15-Anteil wie z.B. ein Fusionsprotein, das eine IL-15-Komponente und eine Immunglobulin-Komponente enthält, handeln (Übersicht in Fehninger und Caligiuri, 2001, Blood 97(1): 14-32). Die Interleukin-Immunglobulin-Fusionsproteine haben sich hierbei als vorteilhaft erwiesen.

Rekombinante Fusionsproteine aus Interleukinen und Immunglobulinen können in prokaryotischen Expressionssystemen hergestellt werden. Ein wesentlicher Nachteil dieser Expressionssysteme ist die fehlende Glykosilierung der prokaryotisch hergestellten Proteine, die zu einer Beeinträchtigung der Funktionalität und Stabilität des exprimierten Produkts führen kann und damit die medizinische Verwendbarkeit der Expressionsprodukte einschränkt. Die Herstellung rekombinanter Fusionsproteine aus Interleukinen und Immunglobulinen in alternativen Expressionssystemen, wie z.B. Säugerzellen, die in der Regel eine korrekte Glykosilierung gewährleisten, ist hingegen mit dem Problem einer vergleichsweise geringen Expressionseffizienz behaftet (Zheng *et al*., 1999, J. Immunol. 163: 4041-4048). Daher besteht ein Bedarf, ein Expressionssystem für Eukaryonten bereitzustellen, mit dem eine Herstellung großer Mengen rekombinanter IL-15/Fc-Fusionsproteine in ausreichender Reinheit möglich ist.

Eine Aufgabe der vorliegenden Erfindung war es somit, ein derartiges, verbessertes Expressionssystem bereitzustellen.

Die Aufgabe wurde gelöst durch die Bereitstellung eines Expressionssystems zur Herstellung eines IL-15/Fc-Fusionsproteins, enthaltend eine oder mehrere Nukleinsäure(n) umfassend
a) mindestens eine Nukleinsäure für ein IL-15/Fc-Fusionsprotein,
b) mindestens einen Promotor und
c) mindestens eine Nukleinsäure für einen CD5-Leader,
wobei der Promotor und die Nukleinsäure für den CD5-Leader funktionsfähig mit der Nukleinsäure für das IL-15/Fc-Fusionsprotein verknüpft sind.

Mit Hilfe des erfindungsgemäßen Expressionssystems ist es möglich, IL-15/Fc-Fusionsproteine z.B. in Eukaryonten mittels rekombinanter DNA-Technologie in größerem Maßstab herzustellen. Damit ermöglicht die vorliegende Erfindung eine Herstellung von IL-15/Fc-Fusionsproteinen für kommerzielle Zwecke.

Unter rekombinanter DNA-Technologie werden im Allgemeinen Technologien zur Übertragung genetischer Informationen z.B. auf Vektoren verstanden. Diese Vektoren ermöglichen eine Weiterverarbeitung der genetischen Information, zum Beispiel durch Einbringung in einen Wirt, was sowohl die Vervielfältigung als auch die Expression der genetischen Information in einer neuen Umgebung ermöglicht. Im Allgemeinen liegt die genetische Information in Form von Nukleinsäuren vor, zum Beispiel in Form von genomischer DNA oder von cDNA, die die Information für ein oder mehrere gewünschte Genprodukte in kodierter Form enthält. Als Vektoren können beispielsweise Plasmide fungieren, in die Nukleinsäuren wie beispielsweise cDNA integriert werden können, um sie zu vervielfältigen und, gegebenenfalls unter Kontrolle transkriptionsregulatorischer Elemente wie z. B. Promotoren, Enhancern oder Silencern, zur Expression in einer Wirtszelle zu bringen. Plasmide können weitere Elemente enthalten, die sowohl die Synthese des gewünschten Expressionsprodukts als auch seine Stabilität und Lokalisierung in der Wirtszelle beeinflussen oder eine Selektionierung des verwendeten Plasmids bzw. Expressionsprodukts ermöglichen.

Als Expressionssystem im Sinne der vorliegenden Erfindung wird eine oder mehrere Nukleinsäure(n) - ggf. in Kombination mit weiteren Elementen, die zur Transkription erforderlich sein können, wie z.B. Ribosomen, Aminosäuren und/oder t-RNAs - bezeichnet, wobei das Expressionssystem die Expression des IL-15/Fc-Fusionsproteins unter geeigneten Bedingungen z.B. in einer geeigneten Wirtszelle bewirken kann.

Gemäß einer bevorzugten Ausführungsform besteht das Expressionssystem aus der/den genannten einen oder mehreren Nukleinsäuren.

Um die Expression in Wirtszellen zu ermöglich, kann/können die Nukleinsäure(n) des Expressionssystems auch ein Bestandteil von einem oder mehreren Vektor(en) sein, welche(r) nach dem Fachmann bekannten Methoden der rekombinanten DNA-Technologie hergestellt werden kann/können (Sambrook *et al*. (Hersg.), 1989, Molecular Cloning: A Laboratory Course Manual. Cold Spring Harbor Press, New York). Dem Fachmann ist eine Vielzahl von Vektoren bekannt, welche im Zusammenhang mit der vorliegenden Erfindung eingesetzt werden können. Für die Expression in eukaryotischen Zellen sind beispielsweise die Hefe-Vektoren pYES (Expression in *S. cerevisiae*; Invitrogen) und pICZ (Expression in *P. pastoris*; Invitrogen) geeignet. Verwendbar sind auch Baculovirus-Vektoren wie pBacPAK9 (Expression in Insektenzellen; Clontech), sowie eine Reihe von Vektoren, die zur heterologen Expression in Säugerzellen eingesetzt werden, wie Rc/CMV, Rc/RSV, pcDNA und weitere SV40-abgeleitete Vektoren, in die neben den zu exprimierenden Nukleinsäuresequenzen geeignete transkriptionsregulatorische Elemente eingefügt werden können.

Geeignete Vektoren enthalten bevorzugt neben einem Replikationsursprung, der die Plasmidreplikation im gewählten Wirt vermittelt, im Allgemeinen selektionierbare Markergene, sowie Erkennungsstellen für Restriktionsendonukleasen, die die Insertion von Nukleinsäurefragmenten ermöglichen. Die für das IL-15/Fc-Fusionsprotein kodierende Nukleinsäure kann über geeignete Erkennungsstellen für Restriktionsendonukleasen in den Vektor eingebracht werden.

Virale Vektorsysteme, die ebenfalls für das erfindungsgemäßen Expressionssystem geeignet sind, umfassen beispielsweise retrovirale, adenovirale, adeno-assoziierte virale Vektoren, sowie Herpesvirus- oder Papillomvirus-Vektoren.

Bei der für ein IL-15/Fc-Fusionsprotein kodierenden Nukleinsäure handelt es sich vorzugsweise um eine DNA oder RNA, besonders bevorzugt um eine genomische DNA, eine cDNA oder Kombinationen davon.

Eine Nukleinsäure für ein IL-15/Fc-Fusionsprotein kodiert für ein IL-15/Fc-Fusionsprotein. Ein IL-15/Fc-Fusionsprotein gemäß der vorliegenden Erfindung ist ein Fusionsprotein, das zwei Fusionsanteile enthält, nämlich eine IL-15-Komponente und eine Fc-Komponente. Rekombinante Proteine, die neben einem funktionellen Protein einen Fusionsanteil eines Immunglobulins enthalten, sind beispielsweise in Capon *et al*. (US 5,428,130) beschrieben.

Bevorzugt handelt es sich um ein Fusionsprotein, das aus einem N-terminalen mutierten oder nicht-mutierten IL-15-Teil und einem C-terminalen Fc-Teil besteht. Solche Proteine sind z.B. in WO 97/41232 und Kim *et al*. (1998, J. Immunol. 160:5742-5748) offenbart.

Der IL-15-Teil des Fusionsproteins vermittelt eine selektive Bindung an den IL-15-Rezeptor (IL-15R), der z.B. auf aktivierten T-Zellen exprimiert ist. Der IL-15-Teil kann daher sowohl ein natürlich vorkommendes IL-15 als auch eine Mutante hiervon sein.

In einer stärker bevorzugten Ausführungsform handelt es sich bei der IL-15-Komponente um das Wildtyp-IL-15. Hierbei kann es sich bei dem IL-15 um ein IL-15 einer jeden Spezies, wie z.B. der Maus, der Ratte, des Meerschweinchens, des Kaninchens, der Kuh, der Ziege, des Schafes, des Pferdes, des Schweins, des Hundes, der Katze oder des Affen, vorzugsweise des Menschen handeln. Eingeschlossen sind auch unterschiedliche Spleißvarianten sowie natürlich auftretende Varianten. Besonders bevorzugt sind hierbei Nukleinsäuren aus Säugetieren, insbesondere die humane oder die murine Form der Nukleinsäuren.

Mutanten des IL-15 schließen IL-15-Komponenten ein, die gegenüber dem natürlich vorkommenden IL-15 eine Mutation aufweisen wie z.B. eine oder mehrere Deletionen, Insertionen oder Substitutionen oder Kombinationen hiervon. Allerdings muss die verwendete IL-15-Variante das IL-15/Fc-Fusionsprotein zur Bindung an den IL-15R befähigen. Dies könnte beispielsweise in einem Radioligandbindungstest unter Verwendung von markiertem IL-15 und Membranen oder Zellen, die IL-15-Rezeptoren aufweisen, überprüft werden (Carson WE et al., 1994, J Exp Med., 180(4): 1395-1403).

In einer bevorzugten Ausführungsform könnte es sich bei der Mutante eine solche Mutante handeln, die eine Wirkung wie IL-15 aufweist (agonistisch wirkende IL-15-Komponente), wobei deren Aktivität im Vergleich zu IL-15 gleich hoch, erniedrigt oder sogar erhöht sein kann. Als Testsystem für IL-15/Fc-Fusionsproteine, die eine agonistisch wirkende IL-15-Komponente besitzen, kann die Stimulation der Proliferation muriner CTLL-2-Zellen durch die IL-15-Komponente verwendet werden.

Eine agonistisch wirkende IL-15-Komponente im Sinne der vorliegenden Erfindung liegt vor, wenn die Komponente mindestens 10 %, vorzugsweise mindestens 25 %, noch weiter bevorzugt um mindestens 50 %, noch stärker bevorzugt 100 %, sogar noch stärker bevorzugt 150 % und am meisten bevorzugt mindestens 200 % Aktivität aufweist. Aktivität einer agonistisch wirkenden IL-15-Komponente meint die prozentuale Stimulation der Antwort durch die IL-15-Komponente im Vergleich zur Stimulation durch Wildtyp-IL-15 (Wildtyp-Il-15 entspricht 100 % Aktivität). In den Tests kann entweder die IL-15-Komponente alleine oder das Fusionsprotein eingesetzt werden.

Für agonistisch wirkende IL-15-Komponenten sind konservative Aminosäureaustausche bevorzugt, wobei ein Rest durch einen anderen mit ähnlichen Eigenschaften ersetzt wird. Typische Substitutionen sind Substitutionen innerhalb der Gruppe der aliphatischen Aminosäuren, innerhalb der Gruppe der Aminosäuren mit aliphatischer Hydroxylseitenkette, innerhalb der Gruppe von Aminosäuren mit sauren Resten, innerhalb der Gruppe der Aminosäuren mit Amidderivaten, innerhalb der Gruppe der Aminosäuren mit basischen Resten oder innerhalb der Aminosäuren mit aromatischen Resten. Typische konservative und halb-konservative Substitutionen sind:

| Aminosäure | Konservative Substitution | Halb-konservative Substitution |
|---|---|---|
| A | G; S; T | N; V; C |
| C | A; V; L | M; I; F; G |
| D | E; N; Q | A; S; T; K; R; H |
| E | D; Q; N | A; S; T; K; R; H |
| F | W; Y; L; M; H | I; V; A |
| G | A | S; N; T; D; E; N; Q |
| H | Y;F;K;R | L; M; A |
| I | V; L; M; A | F; Y; W; G |
| K | R; H | D; E; N; Q; S; T; A |
| L | M; I; V; A | F; Y; W; H; C |
| M | L; I; V; A | F; Y; W; C; |
| N | Q | D; E; S; T; A; G; K; R |
| P | V; I | L; A; M; W; Y; S; T; C; F |
| Q | N | D; E; A; S; T; L; M; K; R |
| R | K; H | N; Q; S; T; D; E; A |
| S | A; T; G; N | D; E; R; K |
| T | A; S; G; N; V | D; E; R; K; I |
| V | A; L; I | M; T; C; N |
| W | F; Y; H | L; M; I; V; C |
| Y | F; W; H | L; M; I; V; C |

In einer weiteren Ausführungsform der vorliegenden Erfindung werden antagonistisch wirkende IL-15-Komponenten eingesetzt. Solche Komponenten hemmen oder inhibieren die Wirkung von IL-15 oder die Bindung von IL-15 an den IL-15R, wobei die Hemmung oder Inhibition vollständig oder nur partiell sein kann. Als Testsystem für IL-15/Fc-Fusionsproteine, die eine antagonistisch wirkende IL-15-Komponente besitzen, kann das in WO 97/41232 beschriebene Testsystem (BAF-BO3-Zellproliferationstest) verwendet werden. Eine antagonistisch wirkende IL-15-Komponente im Sinne der vorliegenden Erfindung liegt vor, wenn die Komponente mindestens 10 %, vorzugsweise mindestens 25 %, noch weiter bevorzugt mindestens 50 % und am meisten bevorzugt mindestens 95 % der IL-15-mediierten Wirkung oder IL-15-Bindung an den IL-15R hemmt oder inhibiert. In den Tests kann entweder die IL-15-Komponente alleine oder das Fusionsprotein eingesetzt werden.

Für antagonistisch wirkende IL-15-Komponenten sind nicht konservative Aminosäureaustausche bevorzugt, wobei ein Rest durch einen anderen mit anderen Eigenschaften ersetzt wird. Weiter bevorzugt finden diese Austausche in Bereichen des Moleküls statt, die für die Wechselwirkung mit dem IL-15-R oder für die Signalweiterleitung verantwortlich sind.

In einer bevorzugten Ausführungsform werden als antagonistisch wirkende IL-15-Komponenten die in WO 97/41232 beschriebenen Mutanten des IL-15 oder eine IL-15-Komponente mit einer Mutation an der Aminosäureposition 56 (Aspartat; AAA21551) verwendet. Höchst bevorzugte Mutanten sind die, bei denen Punktmutationen an den Aminosäurepositionen 149 und/oder 156 des Interleukins-15 von Glutamin zu insbesondere Aspartat eingeführt wurden (siehe WO 97/41232). Die beschriebenen Mutationen können in einer Ausführungsform auch kombiniert werden.

In einer Ausführungsform ist der mutierte IL-15-Teil des Fusionsproteins zu mindestens 65 %, vorzugsweise mindestens 70 %, weiter bevorzugt mindestens 85 %, noch weiter bevorzugt mindestens 95 % und am meisten bevorzugt mindestens 99 % identisch mit dem einem Wildtyp-IL-15, vorzugsweise einem humanen Wildtyp-IL-15 (z.B. Datenbank des National Center for Biotechnology Information, Zugangsnummer AAA21551) oder auch anderen natürlich vorkommenden Varianten (z.B. die Varianten mit den Zugangsnummern CAA63914 oder CAA71044 der Datenbank des National Center for Biotechnology Information).

Als zweite funktionelle Einheit des IL-15/Fc-Fusionsproteins ist eine Fc-Komponente vorhanden. Unter dem Fc-Teil versteht man das durch Papainspaltung darstellbare, in der Aminosäuresequenz stark konservierte, konstante (c = constant) Fragment der Immunglobuline. Das Fc-Fragment ist das Fragment des Antikörpers, das üblicherweise keine Antigene bindet. Unter einem Fc-Teil gemäß der vorliegenden Erfindung wird vorzugsweise auch ein wie oben definiertes Fragment eines Immunglobulins verstanden, das darüber hinaus neben der Hinge-Region auch die konstanten Domänen CH2 und CH3 umfasst.

Die Fc-Komponente ist aus dem Fc-Teil eines beliebigen Antikörpers, z.B. eines IgA, IgD, IgG, IgE oder IgM, vorzugsweise eines IgM oder eines IgG, mehr bevorzugt aus einem Fc-Teil der Subklassen IgG1, IgG2, IgG3 oder IgG4, abgeleitet.

In einer besonderen Ausführungsform der Erfindung handelt es sich bei dem Fc-Teil des Fusionsproteins um ein Fc-Fragment eines Immunglobulins G (IgG), dem die leichten Ketten und die schweren Ketten der IgG-variablen Region fehlen. Beispiele für einsetzbare IgGs sind IgG1, IgG2, IgG2a, IgG2b, IgG3 oder IgG4. Bevorzugt ist humanes oder murines IgG1.

Für die vorliegende Erfindung kann der gesamte Fc-Teil des Antikörpers oder nur ein Teil hiervon verwendet werden. Allerdings sollte der Teil des Fc-Teil vorzugsweise so gestaltet sein, dass das IL-15/Fc-Fusionsprotein eine größere Halbwertszeit für die Zirkulation im Blut besitzt als die IL-15-Komponente ohne Immunglobulin-Komponente. Um dies zu testen, kann einem oder mehreren Versuchstieren das Fusionsprotein und die IL-15-Komponente verabreicht z.B. in die Blutbahn injiziert werden und die Halbwertszeiten für die Zirkulation im Blut verglichen werden. Eine größere Halbwertszeit liegt vor, wenn die Halbwertszeit erhöht ist, vorzugsweise mindestens um 10 %, weiter bevorzugt mindestens um 20 %, noch weiter bevorzugt um mindestens 50 % und am meisten bevorzugt um mindestens 100 %.

Der Fc-Teil kann auch ein Fc-Teil mit mindestens einer Mutation sein. Das mutierte Fc kann in der Weise mutiert sein, wie dies oben für den IL-15-Teil beschrieben ist.

In einer Ausführungsform ist der mutierte Fc-Teil des Fusionsproteins zu mindestens 65 %, vorzugsweise mindestens 70 %, weiter bevorzugt mindestens 85 %, noch weiter bevorzugt mindestens 95 % und am meisten bevorzugt mindestens 99 % identisch mit dem Fc-Teil eines murinen oder humanen Wildtyp-Immunglobulins, vorzugsweise dem human IgG1-Fc oder auch natürlich vorkommenden Varianten.

In einer bevorzugten Ausführungsform der Erfindung liegt der Fc-Anteil des Fusionsproteins in nativer Form oder mit konservativen Aminosäureaustauschen vor und enthält intakte FcR-und/oder Komplement-Bindungsstellen. Der Fc-Anteil des Fusionsproteins kann sowohl die Aktivierung des Komplementsystems, als auch die Bindung an Fc-Rezeptor-exprimierende Zellen vermitteln und damit zur Depletion der durch den IL-15-Anteil des Fusionsproteins erkannten Zellen führen. Durch Einführung von Mutationen, insbesondere von nicht-konservativen Aminosäureaustauschen, an den Aminosäurepositionen, die die Komplementaktivierung und die Fc-Rezeptorbindung vermitteln, können diese Funktionen ausgeschaltet werden. Dies sind beispielsweise Mutationen der Bindungsstelle für den Fc-Rezeptor (FcR) bzw. den Komplementbindungsstellen (an den Aminosäurepositionen 214, 356, 358 und/oder 435 im nativen humanen IgG1 oder Leu 235, Glu 318, Lys 320 und/oder Lys 322 im nativen murinen IgG2A). Werden Aminosäuren an diesen Positionen ausgetauscht, kommt es in der Regel zu einem Verlust der lytischen und komplementaktivierenden Funktion des Fc-Anteils (WO 97/41232).

Noch weiter bevorzugt ist eine Ausführungsform, in der die Aminosäure Cystein an Position 4 der Hinge-Region des humanen Fc-Anteils, weiter bevorzugt des humanen IgG1 (Position 167 von humanem IgG1), gegen Alanin ausgetauscht ist, beispielsweise um eine intermolekulare Brückenbildung und damit Aggregation des exprimierten IL-15/Fc-Fusionsproteins zu verhindern.

In einer anderen bevorzugten Ausführungsform handelt es sich dem Fc-Teil des humanen Immunglobulins IgG1 oder des murinen Immunglobulins IgG2A, der neben der Gelenkregion die Regionen CH2 und CH3 der schweren Kette umfasst.

In dem IL-15/Fc-Fusionsprotein ist die IL-15-Komponente an die Immunglobulin-Komponente entweder direkt oder über einen Linker fusioniert. Vorzugweise besteht der Linker aus höchstens 25 Aminosäuren, weiter bevorzugt aus höchstens 15 Aminosäuren, noch weiter bevorzugt aus höchstens 10 Aminosäuren und am meistens bevorzugt aus 1, 2, 3, 4 oder 5 Aminosäuren.

In einer noch anderen bevorzugten Ausführungsform wird eine humane, für ein Interleukin kodierende Nukleinsäure entweder mit einer ebenfalls humanen, für ein Fc kodierende Nukleinsäure oder mit einer für ein Fc kodierenden Nukleinsäure aus anderen Spezies, wie z.B. Maus oder Ratte, kombiniert. Beispielsweise kann eine humane, für IL-15 kodierende Nukleinsäure mit einer ebenfalls humanen, für IgG1 kodierenden Nukleinsäure, mit einer murinen, für IgG2A kodierenden Nukleinsäure, oder mit einer für IgG2B kodierenden Nukleinsäure aus der Ratte kombiniert werden. Weitere mögliche Kombinationen von Nukleinsäuren sind für den Fachmann ersichtlich.

Die am meisten bevorzugte Nukleinsäure für ein IL-15/Fc-Fusionsprotein ist die Sequenz der Positionen 979 bis 2014 von SEQ ID NR. 1, die der Positionen 1985 bis 3020 von SEQ ID NR. 2 oder SEQ ID NR. 3 oder eine Nukleinsäure, die für die Polypeptide der SEQ ID NR. 4 oder SEQ ID NR. 5 kodiert. Der am meisten bevorzugte Vektor umfassend eine Nukleinsäure für ein IL-15/Fc-Fusionsprotein ist ein Vektor der SEQ ID NR. 1 oder SEQ ID NR. 2.

Umfasst von dem Begriff "Nukleinsäure für ein IL-15/Fc-Fusionsprotein" sind aber auch eine Nukleinsäure, die eine Sequenzidentität von mindestens ca. 60 %, vorzugsweise ca. 75 %, besonders bevorzugt ca. 90 % und insbesondere ca. 95 % zu der in SEQ. ID Nr. 3 angegebenen Nukleotidsequenz oder einer Nukleotidsequenz, die für die Polypeptide der SEQ ID NR. 4 oder SEQ ID NR. 5 kodiert, aufweist, wobei die entsprechenden IL-15/Fc-Fusionsproteine an den IL-15R binden und eine erhöhte Halbwertszeit im Blut gegenüber dem entsprechenden IL-15/Fc-Fusionsprotein ohne Immunglobulinkomponente aufweisen (Testsysteme siehe oben).

Umfasst von dem Begriff "Vektor umfassend eine Nukleinsäure für ein IL-15/Fc-Fusionsprotein" ist auch eine Nukleinsäure, die eine Sequenzidentität von mindestens ca. 60 %, vorzugsweise ca. 75 %, besonders bevorzugt ca. 90 % und insbesondere ca. 95 % zu den in SEQ. ID NR. 1 und SEQ ID NR. 2 angegebenen Nukleotidsequenzen aufweist, wobei die entsprechenden IL-15/Fc-Fusionsproteine an den IL-15R binden und eine erhöhte Halbwertszeit im Blut gegenüber dem entsprechenden IL-15/Fc-Fusionsprotein ohne Immunglobulinkomponente aufweisen (Testsysteme siehe oben).

Das Expressionssystem umfasst weiterhin einen Promotor. Der Promotor und seine Funktionen sind dem Fachmann bekannt. Dieser kann aus z.B. Viren, Bakterien oder Eukaryonten abgeleitet sein. Der Promotor kann die Transkription des zu exprimierenden Gens konstitutiv steuern oder induzierbar sein und damit eine gezielte Regulation der Genexpression ermöglichen. Weiterhin kann der Promotor zell- oder gewebespezifisch sein, d.h. die Expression des Genprodukts auf bestimmte Zelltypen beschränken. Promotoren mit diesen Eigenschaften sind dem Fachmann bekannt. Promotoren, die zur Steuerung der Expression in einer Wirtszelle besonders geeignet sind, sind beispielsweise der ADH2-Promotor für die Expression in Hefe, oder der Polyhedrinpromotor für die Expression in Insektenzellen. Promotoren, die eine starke Expression eines Genprodukts in Säugerzellen vermitteln, sind beispielsweise virale Promotoren viraler Gene, wie der RSV(Rous-Sarcoma-Virus)-Promotor, der SV 40(Simian Virus 40)-Promotor sowie der CMVi/e(Cytomegalovirus Immediate Early Polypeptid)-Promotor. Der CMV-Promotor ist im Zusammenhang mit der vorliegenden Erfindung bevorzugt. Auch eingeschlossen sind Mutationen im CMV-Promotor, wobei die mutierte Sequenz bevorzugt zu 95 %, weiter bevorzugt zu 99 %, homolog zum natürlich vorkommenden CMV-Promotor (Kouzarides et al., 1983, Mol Biol. Med. 1(1): 47-58) ist und/oder die Aktivität der Mutante im Vergleich zum Wildtyp-Promotor bevorzugt 90 bis 110 %, weiter bevorzugt 95 bis 105 % beträgt.

Zusätzlich kann der transkriptionsregulatorische Bereich, insbesondere bei Verwendung des CMV-Promotors, ein oder mehrere Introns, vorzugsweise das Intron A (Chapman *et al*., 1991, Nucleic Acids Res. 19(14): 3979-3986), enthalten. Diese Ausführungsform hat den Vorteil, dass besonders hohe Mengen an IL-15/Fc-Fusionsproteinen erzielt werden können, beispielsweise durch Präsentation geeigneter Bindungsstellen für Transkriptionsfaktoren. Auch eingeschlossen sind Mutationen im Intron A, wobei die mutierte Sequenz bevorzugt zu 80 %, weiter bevorzugt zu 90 % und noch weiter bevorzugt zu 95 %, homolog zu einem natürlich vorkommenden Intron, insbesondere Intron A (Chapman *et al*., 1991, Nucleic Acids Res. 19(14): 3979-3986), ist und/oder die Aktivität der Mutante im Vergleich zum Wildtyp-Intron, insbesondere Intron A, bevorzugt 90 bis 110 %, weiter bevorzugt 95 bis 105 % beträgt.

Ein weiteres Element des erfindungsgemäßen Expressionssystems ist eine Nukleinsäure für einen CD5-Leader, d.h. für die sekretorische Signalsequenz des Lymphocytenantigens CD 5 (Jones *et al*., 1986, Nature 323 (6086): 346-349). Diese sekretorische Signalsequenz vermittelt die Sekretion des Expressionsprodukts in das Kulturmedium der Wirtszelle. Der Nukleinsäure für den CD5-Leader und das IL-15/Fc-Fusionsprotein sind im dem Expressionssystem so angeordnet, dass der Leader die Sekretion des Fusionsproteins vermitteln kann. Vorzugsweise ist der CD5-Leader nach Transkription und Translation im Expressionsprodukt carboxyterminal, genauso bevorzugt aber auch aminoterminal, vom Fusionsprotein lokalisiert.

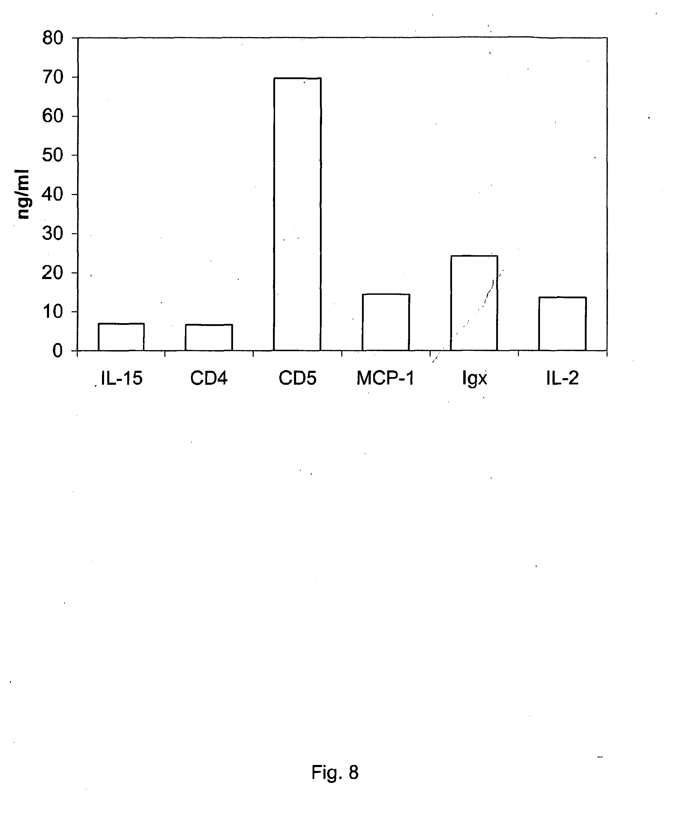

Es zeigte sich überraschenderweise, dass der CD5-Leader in CHO-Zellen eine 200- bis 300-fach höhere Sekretion des Expressionsprodukts ins Zellkulturmedium vermittelt als vergleichbare Signalsequenzen (siehe Beispiel 2, Fig. 8). Auch eingeschlossen sind Mutationen im CD5-Leader, wobei die mutierte Sequenz zu bevorzugt 80 %, weiter bevorzugt zu 90 % und noch weiter bevorzugt zu 95 %, homolog zum natürlich vorkommenden CD5-Leader (Jones *et al*., 1986, Nature 323 (6086): 346-349) ist und/oder die Aktivität der Mutante im Vergleich zum Wildtyp-CD5-Leader bevorzugt 80 bis 120 %, weiter bevorzugt 90 bis 110 % und noch weiter bevorzugt 95 bis 105 % beträgt.

In dem Expressionssystem sind der Promotor und die Nukleinsäure für den CD5-Leader funktionsfähig mit der Nukleinsäure für das IL-15/Fc-Fusionsprotein verknüpft. Unter funktionsfähig verknüpft wird verstanden, dass Promotor und die Nukleinsäure für den Leader in Bezug auf die Nukleinsäure für das Fusionsprotein so angeordnet sind, dass sie ihre Funktion ausüben können. Die Funktion des Promotors besteht in der Regulation der Expression des Fusionsproteins. Wenn beide auf einer Nukleinsäure lokalisiert sind, wird der Promotor sich üblicherweise 5', aber auch 3', vom Fusionsprotein befinden. Die Funktion des Leaders besteht darin, die Sekretion des Fusionsproteins zu vermitteln. Wenn die Nukleinsäure für den Leader und das Fusionsprotein auf einer Nukleinsure lokalisiert sind, wird der Leader üblicherweise das Fusionsprotein flankieren. Bevorzugterweise wird unter "funktionsfähig verknüpft" verstanden, dass der Promotor und der CD5-Leader in Relation zum Fusionsprotein so angeordnet sind, dass der Promotor die Expression des Fusionsproteins reguliert und der CD5-Leader die Sekretion des Fusionsproteins bewirkt.

In einer bevorzugten Ausführungsform enthält das Expressionssystem zusätzlich mindestens eine Nukleinsäure für ein selektionierbares Markergen, das eine Selektion z.B. der mit dem Expressionssystem transfizierten Wirtszelle gegenüber nicht-transfizierten Zellen ermöglicht. Markengene sind z.B. resistenzvermittelnden Gene, die in Kombination mit einem Antibiotikum eingesetzt werden. Dieses Gen wird z.B. in einen Expressionsvektor insertiert und mit einem auf die entsprechend transfizierte Wirtszelle angewandten Antibiotikum eingesetzt. Bekannte zur Selektion eukaryotischer Wirtszellen eingesetzte Antibiotika sind beispielsweise Ampicillin, Kanamycin, Zeocin oder in einer bevorzugten Ausführungsform der Erfindung Neomycin, die eine Selektion von Wirtszellen ermöglichen, indem das entsprechende resistenzvermittelnde Gen exprimiert wird. Dem Fachmann sind weitere Markergene bekannt, bei denen beispielsweise die selektiven Gene tk oder DHFR mit einer Anwendung der entsprechenden selektionierender Agenzien wie HAT bzw. Aminopterin und Methotrexat kombiniert werden. Weitere geeignete selektionierbare Markergene, wie beispielsweise das "Green-Fluorescent-Protein"-Gen aus *A. victoria* und Varianten davon, erlauben eine optische Selektionierung einer mit dem Expressionsvektor transfizierten Wirtszelle, ohne dass die Wirtszelle mit selektionierenden Agenzien behandelt wird.

Bevorzugt wird das für das Enzym Tryptophansynthetase kodierende Gen als selektionierbares Markergen verwendet, wobei das entsprechende Expressionsplasmid zur Selektion und Expression in eine Tryptophansynthetase-defiziente Wirtszelle eingebracht wird.

In einer weiteren bevorzugten Ausführungsform umfasst das Expressionssystem auch mindestens eine Nukleinsäure für ein Polyadenylierungssignal, das üblicherweise neben der Termination der Transkription auch einen Einfluss auf die Stabilität der RNA-Transkripte hat. Beispiele hierfür sind die Polyadenylierungssequenzen aus SV 40, aus dem β-Globin-Gen oder in einer bevorzugten Ausführungsform aus dem bovinen Wachstumshormon-Gen BGH (EP 173552). Die Nukleinsäure für das Polyadenylierungssignal ist in der Art Teil des Expressionssystems, dass es in der Lage ist, die Expression des Fusionsporteins oder seine Stabilität zu verbessern. Üblicherweise ist sie mit der Nukleinsäure für das Fusionsprotein verbunden, so dass das Transkriptionsprodukt die für ein IL-15/Fc-Fusionsprotein kodierende Nukleinsäure und das Polyadenylierungssignal umfasst.

In einer noch weiteren Ausführungsform z.B. für die Transkription und/oder Translation in einem zellfreien System enthält das Expressionssystem zusätzlich zu den oben genannten Bestandteilen Komponenten, die zur Expression benötigt werden. Beispiele für solche mögliche Komponenten sind Transkriptionsfaktoren, Enzyme (z.B. Peptidyl-Transferase, AminoacyltRNS-Synthetase und RNA-Polymerasen) und andere zelluläre Proteine (z.B. eIF4E, eFE1 und eEF2) sowie weitere Hilfsstoffe (ATP, GTP und Magnesiumionen,), vorzugsweise t-RNAs, Aminosäuren und/oder Ribosomen.

In einer bevorzugten Ausführungsform der Erfindung umfasst das Expressionssystem nur eine Nukleinsäure, die die Komponenten a) bis c) und ggf. d), die alle wie oben definiert sind, enthält.

In einer hoch bevorzugten Ausführungsform der Erfindung enthält das Expressionssystem eine Nukleinsäure einer der Sequenzen SEQ ID NR. 1, SEQ ID NR. 2 oder SEQ ID NR. 3 oder eine Nukleinsäure, die für ein Polypeptid der SEQ ID NR. 4 oder SEQ ID NR. 5 kodiert. Umfasst ist aber auch eine Nukleinsäure, die eine Sequenzidentität von mindestens ca. 60 %, vorzugsweise ca. 75 %, besonders bevorzugt ca. 90 % und insbesondere ca. 95 % zu einer der in den SEQ ID NR. 1, SEQ ID NR. 2 oder SEQ. ID Nr. 3 angegebenen Nukleotidsequenzen oder einer Nukleotidsequenz, die für ein Polypeptid der SEQ ID NR. 4 oder SEQ ID NR. 5 kodiert, aufweist, wobei die entsprechenden IL-15/Fc-Fusionsproteine an den IL-15R binden und eine erhöhte Halbwertszeit im Blut gegenüber dem entsprechenden IL-15/Fc-Fusionsproteim ohne Immunglobulinkomponente aufweisen (Testsysteme siehe oben).

In einer höchst bevorzugten Ausführungsform umfasst das Expressionssystem eine Nukleinsäure auf der von 5' nach 3' die folgenden Bestandteile angeordnet sind: CMV-Promotor und ggf. darauf folgend Intron A, CD5-Leader, IL-15/Fc-Fusionsprotein, insbesondere bestehend aus einem IL-15 mit Punktmutationen an den Aminosäurepositionen 149 und/oder 156 des IL-15 von Glutamin zu Aspartat (siehe WO 97/41232) und einem Fc-Teil des humanen IgG1, bei dem der die Aminosäure Cystein an Position 4 der Hinge-Region gegen Alanin ausgetauscht ist, ggf. ein Polyadenylierungssignal und ggf. mindestens ein Markergen. Insbesondere das Markergen kann auch auf einer zweiten Nukleinsäure angeordnet sein. Damit ist auch eine derartige Nukleinsäure (mit oder ohne Markergen) bevorzugte Ausführungsform der erfindungsgemäßen Nukleinsäure.

- Ein weiterer Gegenstand der vorliegenden Erfindung ist eine Nukleinsäure, die das IL-15/Fc-Fusionsprotein, den Promotor, den CD5-Leader, ggf. das selektionierbare Markergen und ggf. das Polyadenylierungssignal umfasst, wobei alle Komponenten wie oben beschrieben sind. In einer bevorzugten Ausführungsform enthält die Nukleinsäure die Sequenz von SEQ ID Nr. 1, SEQ ID Nr. 2 oder 3 oder eine Nukleinsäure, die für ein Polypeptid der SEQ ID NR. 4 oder SEQ ID NR. 5 kodiert. Umfasst ist aber auch eine Nukleinsäure, die eine Sequenzidentität von mindestens ca. 60 %, vorzugsweise ca. 75 %, besonders bevorzugt ca. 90 % und insbesondere ca. 95 % zu einer der in den SEQ ID NR. 1, SEQ ID NR. 2 oder SEQ. ID Nr. 3 angegebenen Nukleotidsequenzen oder einer Nukleotidsequenz, die für ein Polypeptid der SEQ ID NR. 4 oder SEQ ID NR. 5 kodiert, aufweist, wobei die entsprechenden IL-15/Fc-Fusionsproteine an den IL-15R binden und eine erhöhte Halbwertszeit im Blut gegenüber dem entsprechenden IL-15/Fc-Fusionsprotein ohne Immunglobulinkomponente aufweisen (Testsysteme siehe oben).

Ein weiterer Gegenstand der Erfindung ist eine Wirtszelle, die ein erfindungsgemäßes Expressionssystem oder eine erfindungsgemäße Nukleinsäure enthält.

Als Wirtszellen können eukaryotische Zellen wie Hefezellen (z.B. *S*. *cerevisiae, P. pastoris*), Insektenzellen (z.B. Sf9) oder Säugerzellen verwendet werden. Beispiele solcher Säugerzellen sind die humane embryonale Nierenzelllinie HEK-293, die aus Ovarienzellen des chinesischen Hamsters hergestellte Zelllinie CHO und ihre Derivate, wie z.B. CHO-K1 und CHO-DHFR, die Zelllinien BHK, NIH 3T3, HeLa, COS-1, COS-7 oder NS.1. Vorzugsweise handelt es sich um eine Säugerzelle, weiter bevorzugt um eine CHO-Zelle oder deren Derivate, am meisten bevorzugt um eine CHO-K1-Zelllinie.

In einer bevorzugten Ausführungsform handelt es sich bei der Wirtszelle um solche Zellen, die mit der/den Nukleinsäure(n) des Expressionssystems stabil transfiziert sind. Bei stabil transfizierten Zellen wird das Expressionssystem in das Genom der Zielzelle eingebaut und verbleibt stabil im Genom. Das übertragene Gen wird hier im Gegensatz zur transienten Transfektion nicht nur nicht abgebaut, sondern bei jeder Zellteilung verdoppelt und an die Tochterzellen weitergegeben. Diese behalten damit die Fähigkeit, das gewünschte Protein über einen langen Zeitraum hinweg herzustellen.

Verfahren zum Herstellen transfizierter, insbesondere stabil transfizierter, Zellen sind dem Fachmann bekannt. Die Transformation der Wirtszelle kann beispielsweise mittels Elektroporation, bei der durch kurzzeitiges Anlegen eines elektrischen Feldes eine Permeabilisierung der Zellmembran die Aufnahme von Nukleinsäuren in die Zelle erlaubt, oder durch Transfektion oder Infektion mit einem viralen Vektor erfolgen. Neben einer transienten Expression des rekombinanten Proteins kann das verwendete Expressionssystem auch eine klonale Selektionierung der transfizierten Wirtszellen erlauben, so dass klonale Zelllinien mit einer geeigneten Expressionseffizienz selektiert werden können.

In einer bevorzugten Ausführungsform handelt es sich bei der Wirtszelle um eine eukaryotische Säugerzelle, die mindestens eine Nukleinsäure gemäß SEQ ID NR. 1, SEQ ID NR. 2 oder SEQ ID NR. 3 oder eine Nukleinsäure, die für die Polypeptide der SEQ ID NR. 4 oder SEQ ID NR. 5 kodiert, enthält. Umfasst sind auch die Nukleinsäuren, die eine Sequenzidentität von mindestens ca. 60 %, vorzugsweise ca. 75 %, besonders bevorzugt ca. 90 % und insbesondere ca. 95 % zu den genannten Sequenzen aufweisen, wobei die entsprechenden IL-15/Fc-Fusionsproteine an den IL-15R binden und eine erhöhte Halbwertszeit im Blut gegenüber dem entsprechenden IL-15/Fc-Fusionsprotein ohne Immunglobulinkomponente aufweisen (Testsysteme siehe oben).

In einer besonders bevorzugten Ausführungsform handelt es sich bei der erfindungsgemäßen Wirtszelle um eine Zelle der Linie CHO-K1 (Subklon von Ovarienzellen des Chinesischen Hamsters), die mit mindestens einer Nukleinsäure gemäß SEQ ID NR. 1, SEQ ID NR. 2 und/oder SEQ ID NR. 3 oder einer Nukleinsäure, die für die Polypeptide der SEQ ID NR. 4 oder SEQ ID NR. 5 kodiert, stabil transfiziert ist. Umfasst sind auch die Nukleinsäuren, die eine Sequenzidentität von mindestens ca. 60 %, vorzugsweise ca. 75 %, besonders bevorzugt ca. 90 % und insbesondere ca. 95 % zu den genannten Sequenzen aufweisen, wobei die entsprechenden IL-15/Fc-Fusionsproteine an den IL-15R binden und eine erhöhte Halbwertszeit im Blut gegenüber dem entsprechenden IL-15/Fc-Fusionsprotein ohne Immunglobulinkomponente aufweisen (Testsysteme siehe oben).

Ein weiterer Gegenstand der Erfindung ist ein Verfahren zur Herstellung eines wie oben definierten IL-15/Fc-Fusionsproteins, umfassend
a. Bereitstellen einer wie oben beschriebenen Wirtszelle,
b. Kultivieren der Wirtszelle,
c. gegebenenfalls Selektionieren und
d. Isolieren des exprimierten IL-15/Fc-Fusionsproteins.

Als Wirtszellen können beispielsweise die oben beschriebenen Zellen verwendet werden. Vorzugsweise handelt es sich um eine Säugerzelle, weiter bevorzugt um eine CHO-Zelle oder deren Derivate, am meisten bevorzugt um eine CHO-K1-Zelllinie. Die Transfektion einer geeigneten Wirtszelle mit den für das IL-15/Fc-Fusionsprotein kodierenden Nukleinsäuren kann nach Standardmethoden erfolgen (Sambrook *et al*., 1989, supra).

Bei den transfizierten Wirtzellen kann es sich sowohl um adhärente Zellen als auch um eine Suspensionskultur handeln. Vorteilhafterweise liegen die verwendeten Wirtszellen in Suspensionskultur vor, was eine Verringerung der Expressionseffizienz während der Adaptation von adhärenten Zellen zu Suspensionszellen vermeidet.

Die Kultivierung von primären Zellen und Zelllinien kann nach Standardmethoden (Freshney, 1993, Animal Cell Culture: A practical approach, John Wiley & Sons, Inc.) in geeigneten Nährmedien unter Fermentationsbedingungen erfolgen, die den Anforderungen der jeweils verwendeten Wirtszellen hinsichtlich Salzkonzentration, pH-Wert, Vitaminen, Spurenelementen, selektionierenden Agenzien, Temperatur, Belüftung etc. angepasst sind und eine optimale Expression des gewünschten Expressionsprodukts ermöglichen. Vorteilhafterweise werden Nährmedien verwendet, die frei von Serum bzw. Fremdproteinen sind und eine höhere Reinheit des Expressionsproduktes gewährleisten.

Unter Selektionierung, insbesondere klonalem Selektionieren, ist ein Verfahren zu verstehen, bei dem durch schrittweise Vereinzelung Wirtszellen mit gewünschten Eigenschaften vermehrt werden. Vorzugsweise werden mit dem Verfahren der klonalen Selektion solche Wirtszellklone ausgewählt, die eine ausreichende Expressionshöhe und/oder ein hohes Glykosilierungsmuster und Sialysierungsstatus des Expressionsprodukts gewährleisten. Glykosilierungsmuster und Sialysierungsstatus beeinflussen unter anderem die Halbwertzeit, Biodistribution, Immunogenität und das Aufreinigungsverhalten des Expressionsprodukts. Geeignete Verfahren zur Bestimmung des Glykosilierungsmusters und Sialinsäurestatus sind dem Fachmann bekannt und umfassen unter anderem kombinierte enzymatische Spaltungen mit IEF (isoelektrische Fokussierung), sowie HPAEC-PAD (High-performance anion-exchange chromatography with pulsed amperometric detection).

Weiterhin umfasst das erfindungsgemäße Verfahren die Isolierung der heterolog exprimierten IL-15/Fc-Fusionsproteine aus den Wirtszellen bzw. in einer bevorzugten Ausführungsform aus dem Kulturmedium der Wirtszellen. Die Isolierung und ggf. Aufreinigung rekombinanter Polypeptide kann nach dem Fachmann bekannten Methoden erfolgen, die beispielsweise Zelllyse, differentielle Zentrifugation, Fällung, Gelfiltration, Affinitätschromatographie, Ionenaustauschchromatographie, HPLC Reverse-Phase-Chromatographie etc. umfassen. Eine geeignete Methode zur Aufreinigung rekombinanter Proteine ist beispielsweise die Affinitätschromatographie, bei der eine unlösliche Matrix durch chemische Behandlung einen Liganden binden kann. Als Ligand kann jedes Molekül mit einer aktiven chemischen Gruppe dienen, die an die Matrix zu binden vermag. Der Ligand wird im Allgemeinen so gewählt, dass er das aufzureinigende Polypeptid in reversibler Form zu binden vermag. Das aufzureinigende Molekül wird im vorgereinigten Kulturmedium der Wirtszellen unter Bedingungen, die seine Bindung an den Liganden begünstigen, auf die Matrix appliziert, ein nachfolgender Waschschritt bewirkt die Entfernung von ungebundenen Molekülen aus dem Kulturmedium. Die Elution des aufzureinigenden Polypeptids kann durch Auftragen einer Lösung erfolgen, die die Bindung des Polypeptids vom Liganden löst. Ein weiteres geeignetes Verfahren ist die Anionenaustauschchromatographie, bei der die Bindung der aufzureinigenden Polypeptide an die Matrix über einen positiven oder negativen Ladungsüberschuss erfolgen kann. In einer bevorzugten Ausführungsform des erfindungsgemäßen Verfahrens erfolgt die Aufreinigung der Expressionsprodukte zunächst durch Abtrennung des Zellkulturmediums von den Wirtszellen, dem ein Zentrifugations- und/oder Filtrationsschritt zur Entfernung von Zellresten folgen kann. Die Aufreinigung der rekombinanten IL-15/Fc-Fusionsproteine aus dem vorgereinigten Kulturmedium der Wirtszellen wird in einer bevorzugten Ausführungsform mittels einer Kombination von Protein A-Affinitätschromatographie und Anionenaustauschchromatographie durchgeführt, an die sich gegebenenfalls eine Gelfiltration anschließt. Die Charakterisierung der so gewonnenen Expressionsprodukte hinsichtlich Menge, Identität und Reinheit kann anschließend mittels dem Fachmann bekannten Methoden wie BCA, Bestimmung der optischen Dichte, SDS-PAGE, Western Blot, ELISA, Aminosäureanalyse, aminoterminaler Sequenzierung, Fingerprinting (MALDI), Molekulargewichtsbestimmung (HPLC-ESI) etc. vorgenommen werden.

Ein besonders bevorzugtes Verfahren zur Aufreinigung von IL-15/Fc aus einer Zusammensetzung umfasst die folgenden Schritte:
a) Auftragen der Zusammensetzung auf eine Affinitätschromatographiesäule und Eluieren eines ersten IL-15/Fc-Eluats von der Säule;
b) Auftragen des Eluats aus Schritt a) auf eine Anionenaustauscherchromatographiesäule und Eluieren eines zweiten IL-15/Fc-Eluats von der Säule; und
c) Auftragen des Eluats aus Schritt b) auf eine Gelfiltrationssäule und Eluieren eines dritten IL-15/Fc-Eluats von der Säule.

In einer bevorzugten Ausführungsform ermöglicht das erfindungsgemäßen Verfahren die Herstellung eines IL-15/Fc-Fusionsproteins in einer Menge von mindestens 10 pg/(Zelle x Tag), mehr bevorzugt von mindestens 15 pg/(Zelle x Tag).

In einer weiteren bevorzugten Ausführungsform liegt das Protein nach der Aufreinigung in einer Reinheit von mindestens 90 %, mehr bevorzugt mindestens 95 % und am meisten bevorzugt von mindestens 99 %, vor.

Ein weiterer Gegenstand der vorliegenden Erfindung ist Verwendung eines/r wie oben definierten Expressionssystems, Nukleinsäure oder Wirtzelle zur Herstellung eines IL-15-Fc-Fusionsproteins, wobei die Verwendung wie oben beschrieben erfolgt.

Ein noch weiterer Gegenstand der vorliegenden Erfindung ist die Verwendung eines CD5- Leaders, der wie oben definiert ist, zur Expression eines Proteins in CHO-Zellen und ihren Derivaten, insbesondere CHO-K1-Zellen. Überraschenderweise konnte gezeigt werden, dass bei Verwendung des CD5-Leaders die Expression des Proteins bzw. seine Abgabe in den Zellkulturüberstand 200- bis 300-fach gegenüber einer Expression ohne Leader erhöht ist. Darüber hinaus wurde gezeigt, dass der CD5-Leader an diesen Zellen anderen Leadern deutlich überlegen ist (siehe Beispiel 2, Fig. 8). Bei dem Protein kann es sich um ein beliebiges Protein handeln. In einer bevorzugten Ausführungsform wird die Expression des Proteins von einem CMV-Promotor, insbesondere in Kombination mit Intron A, reguliert wird.

Die folgenden Beispiele und Figuren sollen die Erfindung erläutern, ohne sie darauf zu beschränken.

### BESCHREIBUNG DER FIGUREN

- Fig. 1: zeigt eine Karte des Expressionskonstrukts pcDNA3.1hCD5.6Ala7.
- Fig. 2 - 3: zeigt die Sequenz des Expressionskonstrukts pcDNA3.1hCD5.6Ala7 (SEQ ID NR. 1).
- Fig. 4: zeigt eine Karte des Expressionskonstrukts pMG10Ala7.
- Fig. 5 - 6: zeigt die Sequenz des Expressionskonstrukts pMG10Ala7 (SEQ ID NR. 2).

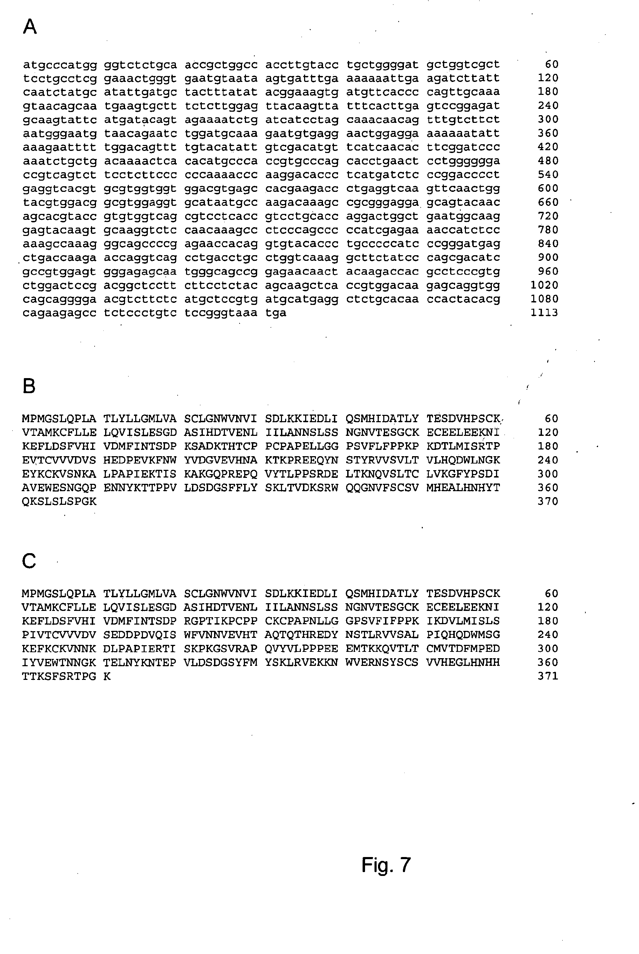

- Fig. 7 A: zeigt die Nukleinsäuresequenz des humanen mutierten IL-15/Fc mit CD5 Leader (SEQ ID NR. 3)
- Fig. 7 B: zeigt die Aminosäuresequenz des humanen mutierten IL-15/Fc mit CD5 Leader (SEQ ID NR. 4)
- Fig. 7 C: zeigt die Aminosäuresequenz des murinen mutierten IL-15/Fc mit CD5 Leader (SEQ ID NR. 5)
- Fig. 8: zeigt den IL-15/Fc-Gehalt in Zellkulturüberständen von CHO-K1-Zellen nach Transfektion mit dem Plasmid pcDNA3.1hCD5.6Ala7, das die jeweils angegebene Leadersequenz enthielt.

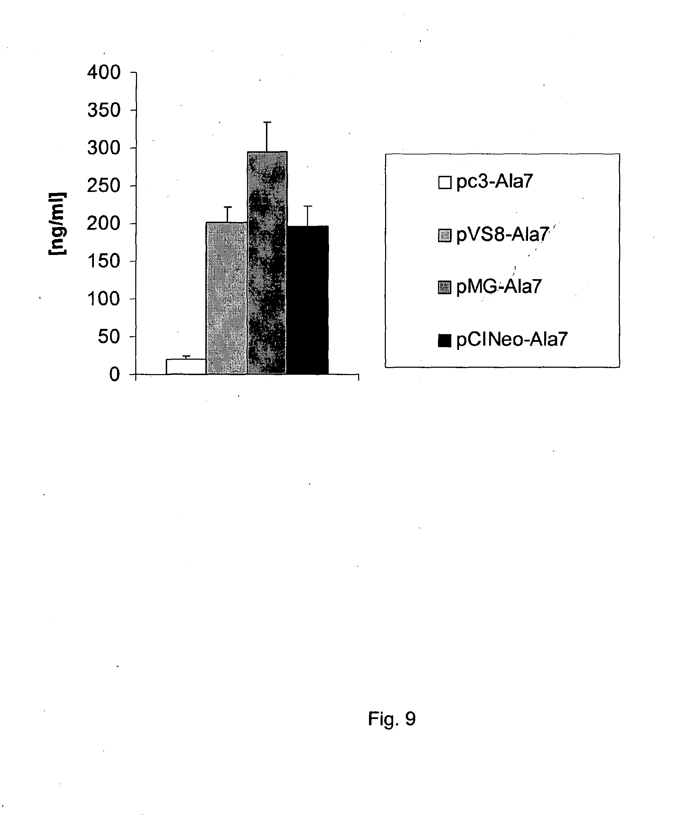

- Fig. 9: zeigt den IL-15/Fc-Gehalt in Zellkulturüberständen von CHO-K1-Zellen nach Transfektion mit verschiedenen Expressionskonstrukten. Jeder Balken repräsentiert den Mittelwert + SEM von Duplikatsbestimmungen von jeweils 2 unabhängigen Experimenten.
pcDNA3.1 entspricht dem Vektor pcDNA3.1hCD5.6Ala7.
pVS8-Ala7 entspricht pSwitch-Plasmid (Valentis) mit dem Konstrukt für IL-15/Fc-Konstrukt.
pMG-Ala7 entspricht pMG-Plasmid (Invivogen) mit dem Konstrukt für IL-15/Fc-Konstrukt.
pCINeo-Ala7 entspricht pCI-Neo-Plasmid (Promega) mit dem Konstrukt für IL-15/Fc-Konstrukt.

### BEISPIELE

### Beispiel 1: Herstellung von IL-15/Fc in CHO-K1-Zellen

Für die Bildung einer CHO-K1-Produzentenzelllinie für IL-15/Fc sollte ein Expressionskonstrukt für IL-15/Fc gebildet und hinsichtlich seiner sekretorischen Eigenschaften, der Identität/Integrität der enthaltenen Fragmente und geeigneter Resistenzgene optimiert werden.

### a) Ausgangsmaterialien

Ein humanes IL-15/Fc-Expressionskonstrukt (mutiertes IL-15/humanes-Fc) wurde von der Immunologieabteilung des "Beth Israel Deaconness Medical Center" (Harvard Medical School, Boston, USA) zur Verfügung gestellt.

Die Oligonukleotide wurden von MWG-Biotech (Ebersberg, Deutschland) erhalten. Die Sequenzen für die relevanten Signalpeptide wurden aus Genbanken erhalten.

Die Restriktionsenzyme (BglII, XBaI, BamHI, SmaI, BstXI, ApaI), Lipfectamin2000, andere molekularbiologische Reagenzien (T4-DNA-Ligase, T4-Polynukleotidkinase) und die Plasmide pSecTagA, pcDNA3.1 wurden von Invitrogen (Karlsruhe, Deutschland) oder Amersham-Pharmacia (NheI, Protein A Sepharose Uppsala, Schweden) erhalten.

Kompetente *E. coli* XL10-Gold-Zellen wurden von Strategene (La Jolla, USA) bezogen. Der BCA-Kit (Pierce) wurde von KMF Laborchemie (Sankt Augustin, Deutschland) erworben.

Die Plasmid-DNA-Reinigungs-Kits (Endofree-Maxi-Kit, Endofree-Giga-Kit) stammten von Qiagen (Hilden, Deutschland).

Die Antikörper wurden von BD-Pharmingen (Maus-anti-hIL-15; Katalognummer 554712; Heidelberg, Deutschland) und Dianova (Ziege-anti-Maus-POD; Katalognummer 15-036-003; Ziege-anti-human-POD; Katalognummer 109-036-088; Hamburg, Deutschland) erhalten.

### b) Methoden/Ergebnisse

Das Ausgangsplasmid enthielt' in dem Vektorgerüst von pSecTagA die cDNA eines Fusionsproteins umfassend ein mutiertes human IL-15, welches an den Fc-Teil (Hinge-Region und CH2-, CH3-Region) des humanen IgG1 fusioniert war. Die Struktur des Plasmid entspricht der von Kim *et al*. beschriebenen (*J. Immunol.,* 160: 5742-5748; 1998) mit der Ausnahme, dass der Fc-Teil, welcher in dieser Veröffentlichung zitiert wird, ein murines Igγ2a ist.

Zur Sekretion des Fusionsproteins wurde der Igk-Leader, der bereits in dem pSecTagA-Vektor enthalten ist, durch Klonieren des IL-15/Fc-Teils im Leserahmen verwendet. Hierfür wurde aus der nativen IL-15-Sequenz die intrinsische Signalsequenz entfernt. Allerdings wurden aufgrund der Klonierung zwischen dem 3'-Ende der Igk-Leadersequenz und dem 5'-Ende der IL-15-kodierenden Sequenz 10 zusätzliche Aminosäuren eingeführt, die nach dem Prozessieren des Proteins beim sekretierten Protein erhalten blieben. Um diese unspezifischen Aminosäuren zu entfernen und die sekretorischen Eigenschaften des Proteins zu verbessern, wurden verschiedene Leadersequenzen anderer sekretorischer oder Zelloberflächenproteine getestet: murines Igk (Coloma *et al., J. Immun. Methods* 152: 89-104; 1992; Zugangsnummer X91670), humanes CD5 (Jones *et al., Nature* 323: 346-349; 1986; Zugangsnummer X04391), CD4 (Hodge *et al., Hum. Immunol*. 30: 99-104; 1991; Zugangsnummer M35160), MCP-1 (Yoshimura *et al. Je. FEBS Lett.* 244: 487-493; 1989; Zugangsnummer M24545) und IL-2 (Taniguchi *et al., Nature* 302: 305-310; 1983; Zugangsnummer K02056) (Zugangsnummern beziehen sich auf das "National Center for Biotechnology Information"). Nach Entfernen des Igk-Leaders und der zusätzlichen Aminosäuren wurde der Leader durch die genannten Signalpeptidsequenzen durch Klonieren von doppelsträngigen Oligonukleotiden ersetzt. Die Identität wurde durch Sequenzieren überprüft. Danach wurden die resultierenden Konstrukte durch transiente Transfektion von HEK-293-Zellen unter Verwendung von Lipfectamin2000 getestet. Der Proteingehalt der Zellkulturüberstände der Zellen, die mit den verschiedenen Konstrukten transfiziert wurden, wurde nach Protein-A-Sepharose-Reinigung nach dem Verfahren von Moll und Vestweber *(Methods in Molecular Biology*, 96: 77-84, 1999) mittels BCA-Test gemessen. Die Identität des Proteins wurde mittels Silberfärbung des SDS-Gels und Western-Blots gegen entweder den Fc- oder den IL-15-Teil überprüft, um die Gegenwart beider Komponenten des Fusionsproteins sicherzustellen. Der CD5-Leader ergab in den beschriebenen Experimenten die besten Ergebnisse und wurde für die weitere Vektoroptimierung ausgewählt.

Weiterhin wurde getestet, ob das Ersetzen der cDNA des Fc-Teils durch die genomische DNA, welche Exon/Intron-Strukturen enthielt, auch zu einer verbesserten Proteinexpression beiträgt. Der RNA-Export aus dem Kern und auch die RNA-Stabilität kann durch das Vorhandensein von Introns verbessert werden, die durch den Splice-Apparat des Kerns entfernt werden müssen. Daher wurde der genomische Fc-Teil an die IL-15-cDNA-Sequenz durch Insertion von Splice-Donor- und -Akzeptorstellen gebunden. Die resultierenden Plasmide wurden ebenfalls durch verschiedene Leadersequenzen modifiziert und wie oben beschrieben getestet. Die Proteinanalyse durch Western-Blot ergab allerdings, dass verschiedene unerwünschte Splice-Varianten vorhanden waren, so dass man sich entschloss, weiterhin die cDNA-Form des Fc-Teils zu verwenden.

Das resultierende Plasmid enthält folglich aus einem humanen CD5-Leader und einem cDNA-Fc-Teil.

Das Sequenzieren des mutierten IL-15/Fc-Expressionskonstrukts ergab, dass 3 Mutationen innerhalb des Fc-Teils vorhanden waren, die schon im Originalkonstrukt vorkamen. Zwei dieser Mutationen betrafen Aminosäuren an hoch konservierten Positionen. Die dritte Mutation war eine Cys-Ala-Mutation an Position 4 in der Hinge-Region, welche absichtlich eingefügt wurde, um die Bildung intra- und intermolekularer Cystein-Brücken zu unterbinden.

Um die zwei unerwünschten Mutationen unter Beibehaltung der Cys-Ala-Mutation zu entfernen, wurde die Fc-cDNA mittels RT-PCR umkloniert. Als RNA-Quelle diente eine CHO-K1-Zelllinie, die mit einem Konstrukt transfiziert war, welches für ein VCAM-1-Fc-Fusionsprotein kodiert. Das amplifizierte Fc-cDNA-Fragment wurde in das CD5-mutIL-15-Plasmid kloniert, wobei der Fc-Teil durch BamHI/XbaI-Restriktion entfernt wurde.

Das resultierende Plasmid wurde wiederum anhand distinkter Restriktionsmuster und mittels anschließenden Sequenzierens analysiert und CD5-6Ala7 genannt. Da die Verwendung von Zeocin als DNA-interkalierendes Agens Mutationen verursachen könnte, wurde die Expressionskassette für IL-15/Fc aus dem ursprünglichen pSecTagA-Gerüst entfernt und in pcDNA3.1 kloniert, welches das Neomycin-Resistenzgen unter der Kontrolle des SV40-Promotors enthält. Beide Stränge des resultierenden Plasmids wurden sequenziert und ergaben eine vollständige Übereinstimmung mit der IL-15/Fc-Expressionskassette.

Das Konstrukt wurde wiederum auf seine Proteinexpression mittels transienter Transfektion von CHO-K1-Zellen und Western-Blot-Analysen des Zellkulturüberstands getestet. Als positive Kontrolle wurde parallel hierzu eine Transfektion mit dem Plasmid CD5-6Ala7 durchgeführt.

Hierzu wurden die Zellen einen Tag vor der Transfektion mit einer Dichte von 5 x 10⁵ Zellen pro Vertiefung in Gewebekulturplatten mit 6 Vertiefungen in Triplikaten ausgesät. Zur Transfektion wurden 2 µg Plasmid und 4 µl Lipofectamin2000, welche jeweils in 250 µl Optimem1-Medium verdünnt wurden, verwendet. Beide Lösungen wurden gemischt und nach 30 min Inkubation bei Raumtemperatur wurde das Gemisch in die Kulturmedien der Gewebekulturplatten pipettiert.

2 Tage nach der Transfektion wurde das Kulturmedium entfernt und auf seinen IL-15/Fc-Gehalt durch einen Western-Blot gegen den humanen IL-15-Teil analysiert: 20 µl des Zellkulturüberstands wurden mit 5 µl 5 x Laemmli-Puffer gemischt und bei 85°C für 5 min inkubiert. Dann wurden die Proben über ein 12% Polyacrylamidgel laufen gelassen. Danach wurde das Gel unter Verwendung einer halbtrockenen Blotkammer geblottet. Der Blot wurde über Nacht mit Blockerlösung enthaltend 5% Milchpulver in PBS, 0,1% Tween20 behandelt. Danach wurde der Blot für 4 Stunden mit einem monoklonalen Maus-anti-humanes-IL-15-Antikörper in einer 1:1000-Verdünnung in Blockerlösung inkubiert. Nach 3 Waschschritten (10 min PBS, 0,1% Tween20) wurde der Blot mit dem Zweitantikörper Ziege-gegen-Maus-Peroxidase (Verdünnung 1:5000) für weitere 2 Stunden bei Raumtemperatur inkubiert. Der Blot wurde wiederum 3 Mal gewaschen und danach Lumilight-Lösung auf die Oberfläche des Blots getropft und ein Röntgenfilm wurde dem Blot ausgesetzt.

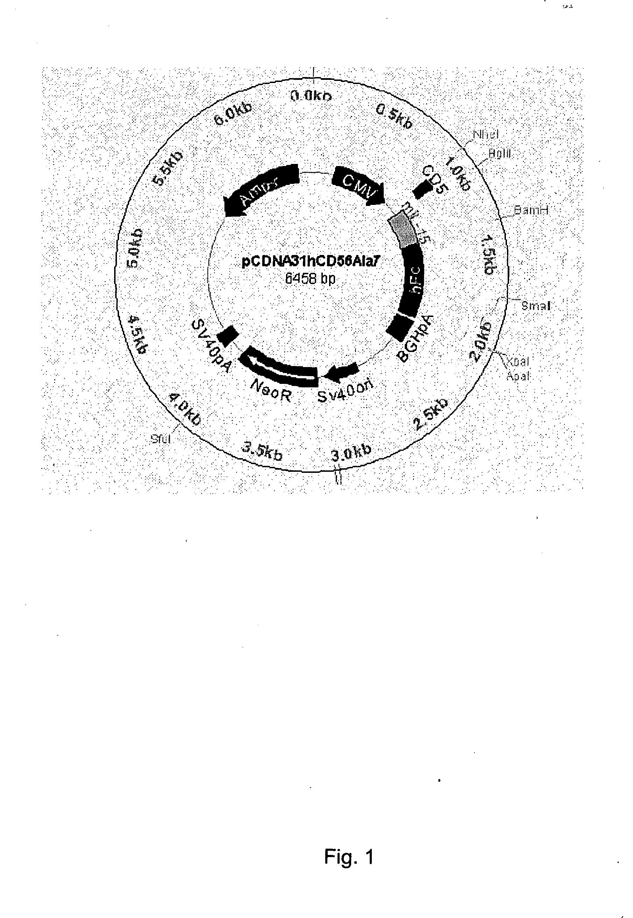

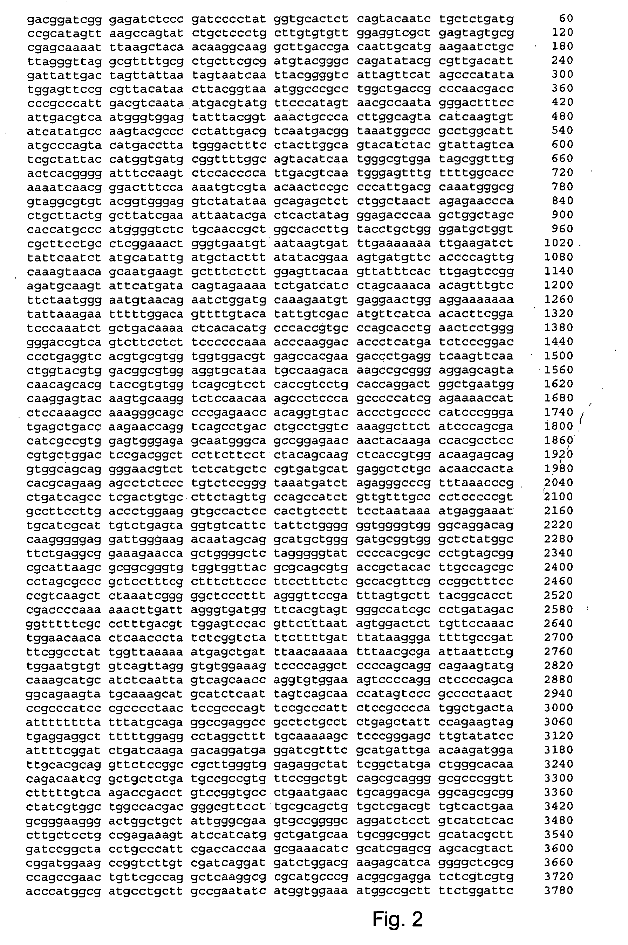

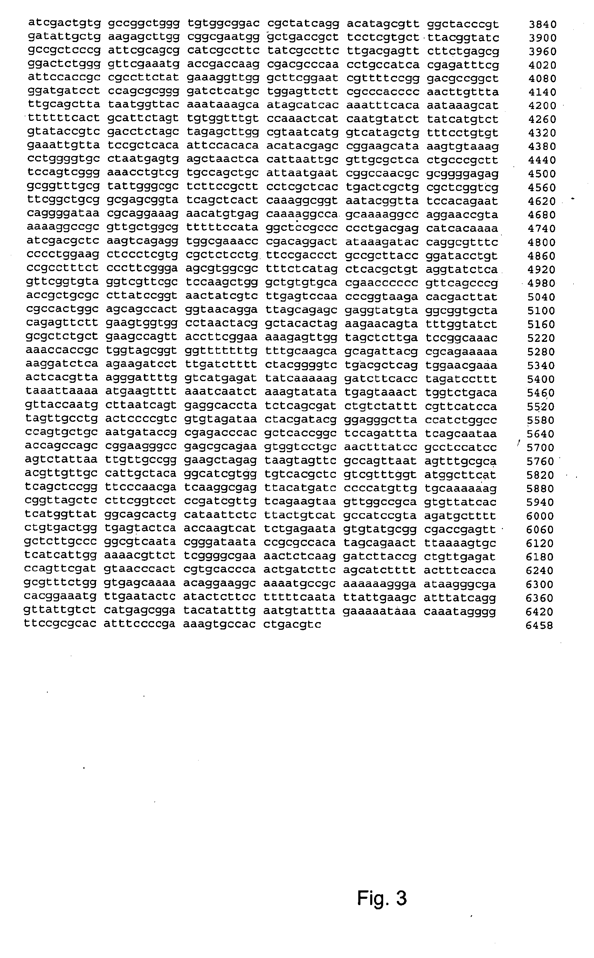

Spezifische Western-Blot-Signale im Bereich der Signale, die nach Transfektion mit CD5-6Ala7 erhalten wurden, ergaben, dass die Zellkulturüberstände aller 3 Paralleltransfektionen IL-15/Fc als Protein enthielten. Es konnte daher gezeigt werden, dass das Plasmid pcDNA3.1hCD5.6Ala7 (Fig. 1 bis 3) zur Proteinexpression in CHO-K1-Zellen verwendet werden kann.

### c) Schlussfolgerungen

Ein IL-15/Fc-Plasmid pcDNA3.1hCD5.6Ala7 wurde hergestellt, welches eine Expressionskassette enthielt, die einen CD5-Leader mit einem mutierten humanen IL-15, welches an die cDNA von humanem IgG1-Fc unter der Kontrolle des CMV-Promotors fusioniert war, enthielt. Zur Selektion von stabilen eukaryotischen Zellklonen wurde ein Neomycin-Resistenzgen eingeführt. Das Plasmid wurde sequenziert und ergab eine 100%-ige Übereinstimmung in den relevanten kodierenden Bereichen mit nur einer geringen Diskrepanz (Wiederholung von 3 Basenpaaren) ohne irgendeine Relevanz in dem Vektorgerüst. Die Funktionalität des Konstrukts wurde durch transiente Transfektion von CHO-K1-Zellen überprüft.

### BEISPIEL 2

Die Transfektion eukaryotischer Zelllinien (z.B. CHO-K1-Zellen) mit einem Plasmid, das die DNA für das gewünschte Produkt enthält, ist ein Standardverfahren zur Produktion therapeutischer Proteine. Dennoch sind die niedrigen Produktivitätsspiegel der so hergestellten stabilen Zellklone ein gemeinhin bekanntes Problem. Daher gibt es verschiedene Strategien zur Erhöhung der Produktivität einer bestehenden Zelllinie. Abgesehen von dem Versuch, die Zahl der Plasmidkopien in der Zelle zu erhöhen (z.B. durch das Methotrexat/DHFR-System), besteht weiterhin die Möglichkeit, das Expressionskonstrukt selbst zu modifizieren. Zusätzlich zu einem starken Promotor (z.B. dem CMV-Promotor) führt das Einführen eines Introns möglicherweise zu einer besseren RNA-Stabilität und einem besseren Export der RNA aus dem Kern, welcher von dem Splice-Apparat der Zelle durchgeführt wird. Dennoch muss getestet werden, welche Kombination von Intron/Transgen geeignet ist. Hierfür wurden verschiedene Introns mit dem humanen IL-15-Fc kombiniert, um eine Kombination zu finden, die die IL-15-Fc-Produktion von CHO-K1-Zellen erhöht.

### a) Materialien

Als Plasmid wurde das Plasmid pcDNA3.1hcD5.6Ala7 als Ausgangsplasmid verwendet. Es ist schematisch in Figur 1 dargestellt. Seine Sequenz ist als SEQ ID NR. 1 offenbart.

Als Testsystem wurden sowohl CHO-K1-Zellen (DSM, Braunschweig, Deutschland, Zugangsnummer: ACC110) als auch HEK-293-Zellen (Qbiogene, Grünberg, Deutschland, AE80503, QBI-293A) verwendet. Weiterhin wurden *E. coli*-Zellen (XL10-Gold, Strategene, La Jolla, USA) eingesetzt. Die Zellen wurden unter Standardkulturbedingungen (5% CO₂, 37°C, Feuchtklima) kultiviert. Die CHO-K1-Zellen wurden zwei Mal pro Woche im Verhältnis 1:20 und die HEK-293-Zellen im Verhältnis 1:6 passagiert. Als Medium wurden für die CHO-K1-Zellen DMEM-F12+10% FKS+1% PEN/Strep und für die HEK-293-Zellen DMEM+Glutamax+10% FKS+1% PEN/Strep eingesetzt. Zur Transfektion wurde Optimem1-Medium verwendet. Alle Medien wurden von Invitrogen, Karlsruhe, Deutschland (Katalognummern 31331-028; 32430-027; 51985-018) eingesetzt. Als Plasmid wurde ein pCl-Neo (Promega) mit einem CMV-Promotor und einem chimären Intron, einer 5'-Splice-Donorstelle des humanen beta-Globin-Gens sowie einer 3'-Splice-Akzeptorstelle der IgG schweren Kette der variablen Region eingesetzt. pMG (Invivogen) ist ein prolongierter CMV-Promotor mit einem IntronA aus CMV. pSwitch (Valentis) ist ein synthetisches Intron IVS8. Weiterhin wurden die folgenden Enzyme bzw. Restriktionsenzyme verwendet: ApaI, EcoRV, XbaI, NruI, PacI, SmaI, XhoI, T4-DNA-Ligase, T4-DNA-Polymerase, alkaline Phosphatase aus Kälberinnereien. Diese und andere molekularbiologische Reagenzien (Lipofectamin2000) wurden von Invitrogen erhalten. NheI wurden von Amersham-Pharmacia (Uppsala, Schweden) und die Plasmidreinigungs-Kits von Qiagen, Hilden, Deutschland, erhalten. Das "Expand High Fidelity PCR-System" (Katalognummer 1 732 641) wurde von Roche, Mannheim, Deutschland, erhalten.

### b) Methoden

i) Das IL-15/Fc-Insert aus dem Plasmid pcDNA3.1hCD5.6Ala7 wurde durch NheI/ApaI- Verdau isoliert. Zuerst wurde das Plasmid durch eine ApaI-Restriktion linearisiert und die 5'-Überhänge durch T4-Polymerasebehandlung stumpfendig gemacht. Danach wurde das IL-15/Fc-Insert durch einen anschließenden NheI-Verdau isoliert. Das Fragment wurde mit pcI Neo, welches mit NheI und SmaI verdaut worden war, ligiert.
ii) Der CMV-Promotor von pcDNA3.1hCD5.6Ala7 wurde entfernt und durch den verlängerten CMV-Promotor mit Intron A, welcher aus pMG stammte, ersetzt: Das pMG-Plasmid wurde mit PacI geschnitten und die' Überhänge mittels T4- Polymerasebehandlung stumpfendig gemacht. Nach einer zweiten XbaI-Behandlung wurde das so erhaltene 1,7 kb Fragment, welches den CMV-Promotor + Intron A enthielt, durch Agarosegelelektrophorese gereinigt. Mittels NheI- und anschließendem NruI-Restriktionsverdau wurde der CMV-Promotor aus pcDNA3.hCD5.6Ala7 entfernt. Das resultierende Fragment wurde bei 4°C über Nacht mit dem pMG-Promotor-Intron ligiert.
iii) Das Intron IVS8 wurde mittels PCR amplifiziert und zwischen das 3'-Ende des CMV- Promotors und das 5-Ende des IL-15-Inserts in pcDNA3.1hCD5.6Ala7 kloniert. Das Plasmid wurde mittels NheI-Restriktionsverdau linearisiert und anschließend mit alkaliner Phosphatase aus Kälbereingeweiden behandelt. Das Intron wurde durch PCR mit Primern, die XbaI-Restriktionsschnittstellen enthielten, unter Verwendung des "Expand High Fidelity PCR-Systems" unter den folgenden Bedingungen amplifiziert: Als Reaktionsgemisch wurden 2 µl dNTPs (Qiagen, Taq core kit, 2 mmol/l jedes), 25 pmol Primer, 5 µl 10 x Puffer, 0,75 µl High Fidelity Taq Polymerase, 1 µl (ungefähr 15 ng) pSwitch-XhoI/EcoRV-Fragment ad 50 µl Wasser verwendet. Das PCR-Programm (25 Zyklen) war wie folgt: 5 min 95°C, 15 sec 94°C, 30 sec 55°C, 30 sec 72°C, 5 min 72°C. Das PCR-Produkt wurde mit XbaI geschnitten, aus einem 0,8% Agarosegel eluiert und mit dem linearisierten Plasmid ligiert.

Die resultierenden Plasmide wurden in *E. coli* XL10 Gold transformiert und die Plasmide mittels Miniprep analysiert. Ein Klon eines jeden Plasmids, welches ein geeignetes Restriktionsmuster zeigte, wurde für die anschließende Endotoxin-freie Plasmidpräparation verwendet.

Die IL-15/Fc-Expression wurde nach transienter Transfektion von HEK-293- oder CHO-K1-Zellen analysiert. Einen Tag vor Durchführung der Transfektion wurden die Zellen in einer Dichte von 5 x 10⁵ Zellen pro Vertiefung in Zellkulturplatten mit sechs Vertiefungen in Duplikaten ausgesät. Zur Transfektion entsprechend Felgner et al. (Proc. Natl. Acad. Sci. USA, 84:7413-7417; 1987) wurden 2 µg Plasmid und 4 µl Lipofectamin2000 jeweils in 250 µl Optimem1-Medium verdünnt. Beide Lösungen wurden gemischt und nach 30 Minuten Inkubation bei Raumtemperatur wurde das Gemisch in das Zellkulturmedium in den Zellkulturplatten pipettiert. Zwei Tage nach der Transfektion wurde das Kulturmedium entfernt und auf den IL-15/Fc-Gehalt durch einen gegen den Fc-Teil von IL-15/Fc gerichteten ELISA-Test bestimmt.

### c) Ergebnisse

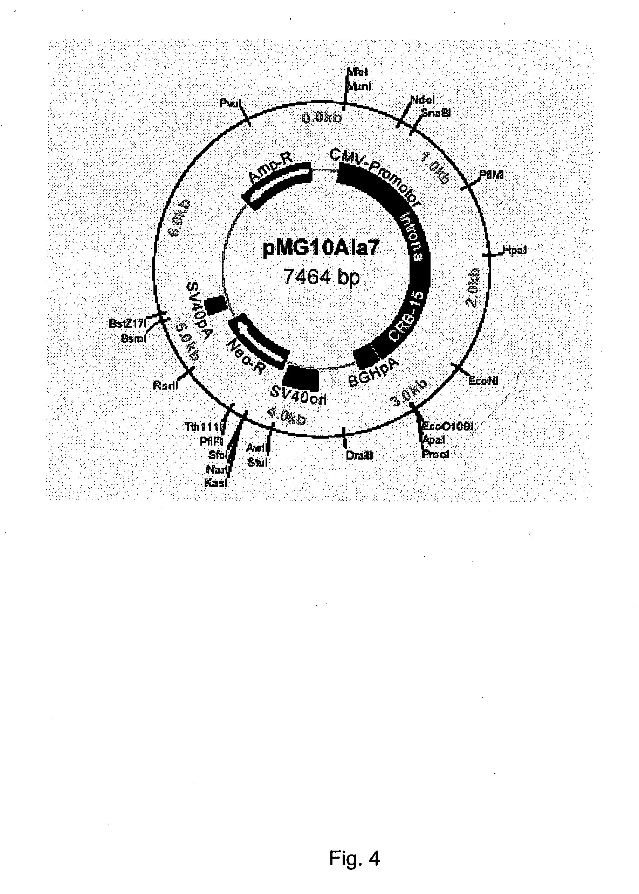

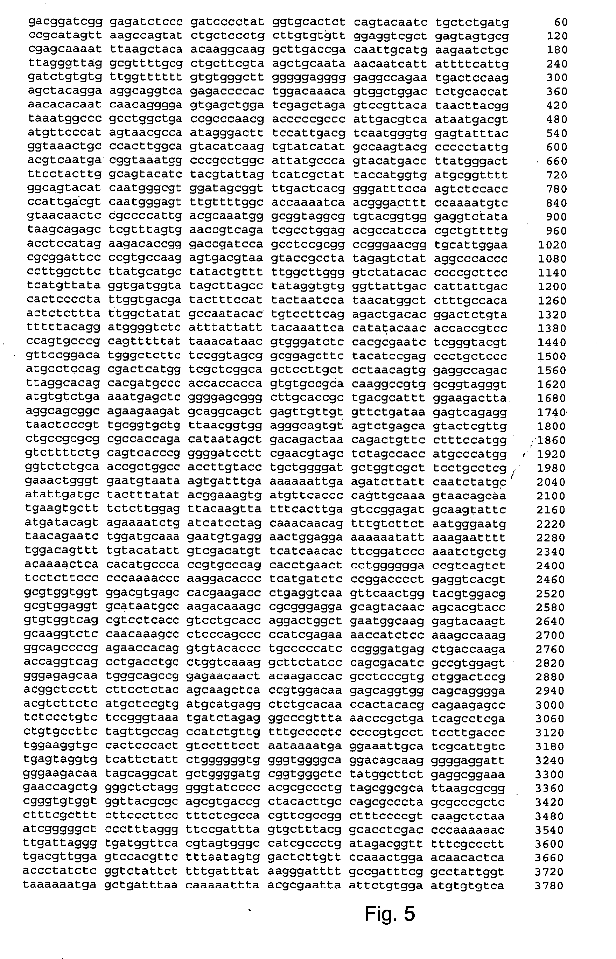

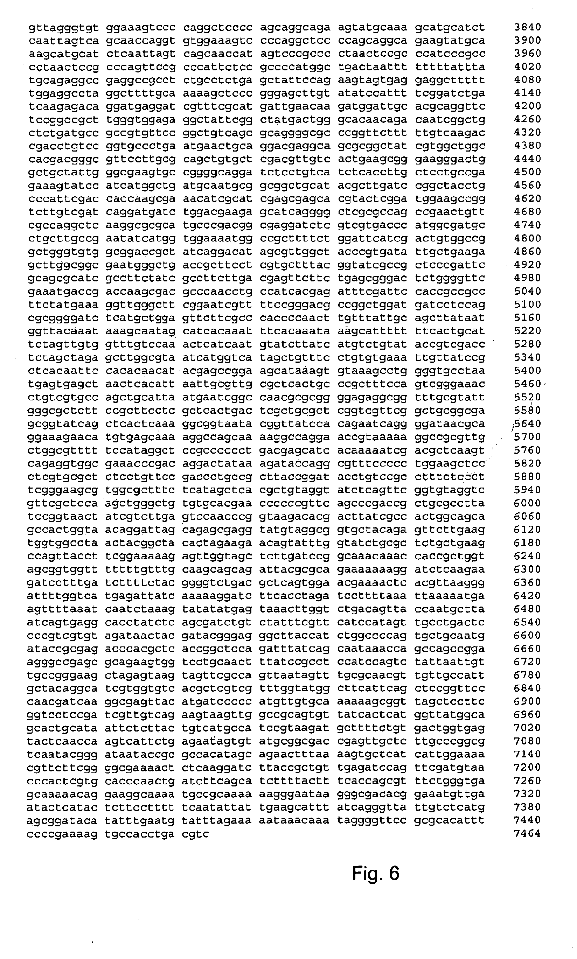

Die IL-15/Fc-Sekretion von HEK-293-Zellen, die mit verschiedenen Expressionskonstrukten transfiziert waren, wurden durch andere Vektorkomponenten kaum beeinflusst. Im Gegensatz dazu war die IL-15/Fc-Expression von CHO-K1-Zellen um den Faktor 200-300 nach der Insertion eines Introns in das IL-15/Fc-Konstrukt erhöht. Das Originalkonstrukt pcDNA3.1hCD5.6Ala7 führte zu Proteinsekretionsspiegeln, die kaum nachweisbar waren (unter 10 ng/ml), wobei die Insertion eines Introns zu IL-15/Fc-Spiegeln von ungefähr 300 ng/ml nach Transfektion der Zellen mit pMG10Ala7 (Fig. 4 bis 6; SEQ ID NR. 2) führte. Die ELISA-Daten, die die IL-15/Fc-Expressionsspiegel in CHO-K1-Zellen zeigen, sind in Figur 4 dargestellt. Da die Expressionsspiegel nach Transfektion mit dem pMG-Konstrukt am höchsten waren, wurde dies für die Bildung einer stabilen CHO-K1-Expressionszelllinie ausgewählt.

Hierzu wurde das Plasmid zunächst einer Einzelstrangsequenzierung unterzogen. Beide Stränge des Konstrukts wurden in dem Bereich, der die IL-15/Fc-Kassette, den neu insertierten CMV-Promotor und das Intronfragment enthielt, sequenziert. Das Plasmid enthielt die IL-15/Fc-Kassette unter der Kontrolle des CMV-Promotors. Zwischen dem Promotor und dem Translationsstart wurde das Intron A, welches aus CMV (Plasmid MG) stammte, positioniert. Das Plasmid enthielt stromabwärts des IL-15/Fc-Fragments eine BGHpolyA-Stelle; das Neomycin-Resistenzgen wurde von einem SV40-Promotor gesteuert und enthielt auch eine SV40polyA-Stelle. Zur Selektion und Amplifikation in *E. coli* enthielt das Plasmid ein Ampicillin-Resistenzgen.

### d) Diskussion und Schlussfolgerungen

Um den Proteinertrag der stabilen CHO-K1-IL-15/Fc-Transfektanten zu erhöhen, wurde das Expressionsplasmid durch Einführen eines Introns zwischen dem Promotor und der IL-15/Fc-Kassette modifiziert. Die Kombination Intron-Transgen-Wirtszelle hat einen großen Einfluss auf die Proteinexpression, weshalb es nicht vorhersagbar ist, welches Intron am wirksamsten in der Steigerung der IL-15/Fc-Expression in den beiden analysierten Zelltypen ist.

Während HEK-293-Zellen von der Einführung des Introns kaum beeinflusst wurden, wurde in CHO-K1-Zellen eine große Erhöhung der IL-15/Fc-Sekretion nachgewiesen. Die Expression des IL-15/Fc-Proteins in CHO-K1-Zellen wurde über weit mehr als eine Größenordnung im Vergleich zum ursprünglichen IL-15/Fc-Expressionsvektor erhöht, wobei ein Plasmid verwendet wurde, das den CMV-Promotor und Intron A aus pMG enthielt. Das Plasmid kann zur Herstellung einer IL-15/Fc-Produzentenzelllinie verwendet werden, die zur Herstellung von IL-15/Fc für vorklinische und klinische Studien oder auch zur industriellen Produktion von IL-15/Fc eingesetzt werden kann.

C62387.ST25.txt
SEQUENCE LISTING

<110> F. Hoffmann-La Roche AG

<120> Expressionssystem zur Herstellung von IL-15/Fc-Fusionsproteinen und ihre Verwendung

<130> C62387EP

<160> 5

<170> PatentIn version 3.1

<210> 1
<211> 6458
<212> DNA
<213> Artificial sequence

<220>
<223> Plasmid pcDNA3.1hCD5.6Ala7

<400> 1
gacggatcgg gagatctccc gatcccctat ggtgcactct cagtacaatc tgctctgatg 60
ccgcatagtt aagccagtat ctgctccctg cttgtgtgtt ggaggtcgct gagtagtgcg 120
cgagcaaaat ttaagctaca acaaggcaag gcttgaccga caattgcatg aagaatctgc 180
ttagggttag gcgttttgcg ctgcttcgcg atgtacgggc cagatatacg cgttgacatt 240
gattattgac tagttattaa tagtaatcaa ttacggggtc attagttcat agcccatata 300
tggagttccg cgttacataa cttacggtaa atggcccgcc tggctgaccg cccaacgacc 360
cccgcccatt gacgtcaata atgacgtatg ttcccatagt aacgccaata gggactttcc 420
attgacgtca atgggtggag tatttacggt aaactgccca cttggcagta catcaagtgt 480
atcatatgcc aagtacgccc cctattgacg tcaatgacgg taaatggccc gcctggcatt 540
atgcccagta catgacctta tgggactttc ctacttggca gtacatctac gtattagtca 600
tcgctattac catggtgatg cggttttggc agtacatcaa tgggcgtgga tagcggtttg 660
actcacgggg atttccaagt ctccacccca ttgacgtcaa tgggagtttg ttttggcacc 720
aaaatcaacg ggactttcca aaatgtcgta acaactccgc cccattgacg caaatgggcg 780
gtaggcgtgt acggtgggag gtctatataa gcagagctct ctggctaact agagaaccca 840

Seite 1

C62387.ST25.txt
ctgcttactg gcttatcgaa attaatacga ctcactatag ggagacccaa gctggctagc 900
caccatgccc atggggtctc tgcaaccgct ggccaccttg tacctgctgg ggatgctggt 960
cgcttcctgc ctcggaaact gggtgaatgt aataagtgat ttgaaaaaaa ttgaagatct 1020
tattcaatct atgcatattg atgctacttt atatacggaa agtgatgttc accccagttg 1080
caaagtaaca gcaatgaagt gctttctctt ggagttacaa gttatttcac ttgagtccgg 1140
agatgcaagt attcatgata cagtagaaaa tctgatcatc ctagcaaaca acagtttgtc 1200
ttctaatggg aatgtaacag aatctggatg caaagaatgt gaggaactgg aggaaaaaaa 1260
tattaaagaa tttttggaca gttttgtaca tattgtcgac atgttcatca acacttcgga 1320
tcccaaatct gctgacaaaa ctcacacatg cccaccgtgc ccagcacctg aactcctggg 1380
gggaccgtca gtcttcctct tccccccaaa acccaaggac accctcatga tctcccggac 1440
ccctgaggtc acgtgcgtgg tggtggacgt gagccacgaa gaccctgagg tcaagttcaa 1500
ctggtacgtg gacggcgtgg aggtgcataa tgccaagaca aagccgcggg aggagcagta 1560
caacagcacg taccgtgtgg tcagcgtcct caccgtcctg caccaggact ggctgaatgg 1620
caaggagtac aagtgcaagg tctccaacaa agccctccca gcccccatcg agaaaaccat 1680
ctccaaagcc aaagggcagc cccgagaacc acaggtgtac accctgcccc catcccggga 1740
tgagctgacc aagaaccagg tcagcctgac ctgcctggtc aaaggcttct atcccagcga 1800
catcgccgtg gagtgggaga gcaatgggca gccggagaac aactacaaga ccacgcctcc 1860
cgtgctggac tccgacggct ccttcttcct ctacagcaag ctcaccgtgg acaagagcag 1920
gtggcagcag gggaacgtct tctcatgctc cgtgatgcat gaggctctgc acaaccacta 1980
cacgcagaag agcctctccc tgtctccggg taaatgatct agagggcccg tttaaacccg 2040
ctgatcagcc tcgactgtgc cttctagttg ccagccatct gttgtttgcc cctcccccgt 2100
gccttccttg accctggaag gtgccactcc cactgtcctt tcctaataaa atgaggaaat 2160
tgcatcgcat tgtctgagta ggtgtcattc tattctgggg ggtggggtgg ggcaggacag 2220
caagggggag gattgggaag acaatagcag gcatgctggg gatgcggtgg gctctatggc 2280
ttctgaggcg gaaagaacca gctggggctc tagggggtat ccccacgcgc cctgtagcgg 2340
cgcattaagc gcggcgggtg tggtggttac gcgcagcgtg accgctacac ttgccagcgc 2400
cctagcgccc gctcctttcg ctttcttccc ttcctttctc gccacgttcg ccggctttcc 2460
ccgtcaagct ctaaatcggg ggctcccttt agggttccga tttagtgctt tacggcacct 2520
cgaccccaaa aaacttgatt agggtgatgg ttcacgtagt gggccatcgc cctgatagac 2580
ggtttttcgc cctttgacgt tggagtccac gttctttaat agtggactct tgttccaaac 2640
tggaacaaca ctcaacccta tctcggtcta ttcttttgat ttataaggga ttttgccgat 2700
ttcggcctat tggttaaaaa atgagctgat ttaacaaaaa tttaacgcga attaattctg 2760
tggaatgtgt gtcagttagg gtgtggaaag tccccaggct ccccagcagg cagaagtatg 2820
caaagcatgc atctcaatta gtcagcaacc aggtgtggaa agtccccagg ctccccagca 2880
Seite 2

C62387.ST25.txt
ggcagaagta tgcaaagcat gcatctcaat tagtcagcaa ccatagtccc gcccctaact 2940
ccgcccatcc cgcccctaac tccgcccagt tccgcccatt ctccgcccca tggctgacta 3000
atttttttta tttatgcaga ggccgaggcc gcctctgcct ctgagctatt ccagaagtag 3060
tgaggaggct tttttggagg cctaggcttt tgcaaaaagc tcccgggagc ttgtatatcc 3120
attttcggat ctgatcaaga gacaggatga ggatcgtttc gcatgattga acaagatgga 3180
ttgcacgcag gttctccggc cgcttgggtg gagaggctat tcggctatga ctgggcacaa 3240
cagacaatcg gctgctctga tgccgccgtg ttccggctgt cagcgcaggg gcgcccggtt 3300
ctttttgtca agaccgacct gtccggtgcc ctgaatgaac tgcaggacga ggcagcgcgg 3360
ctatcgtggc tggccacgac gggcgttcct tgcgcagctg tgctcgacgt tgtcactgaa 3420
gcgggaaggg actggctgct attgggcgaa gtgccggggc aggatctcct gtcatctcac 3480
cttgctcctg ccgagaaagt atccatcatg gctgatgcaa tgcggcggct gcatacgctt 3540
gatccggcta cctgcccatt cgaccaccaa gcgaaacatc gcatcgagcg agcacgtact 3600
cggatggaag ccggtcttgt cgatcaggat gatctggacg aagagcatca ggggctcgcg 3660
ccagccgaac tgttcgccag gctcaaggcg cgcatgcccg acggcgagga tctcgtcgtg 3720
acccatggcg atgcctgctt gccgaatatc atggtggaaa atggccgctt ttctggattc 3780
atcgactgtg gccggctggg tgtggcggac cgctatcagg acatagcgtt ggctacccgt 3840
gatattgctg aagagcttgg cggcgaatgg gctgaccgct tcctcgtgct ttacggtatc 3900
gccgctcccg attcgcagcg catcgccttc tatcgccttc ttgacgagtt cttctgagcg 3960
ggactctggg gttcgaaatg accgaccaag cgacgcccaa cctgccatca cgagatttcg 4020
attccaccgc cgccttctat gaaaggttgg gcttcggaat cgttttccgg gacgccggct 4080
ggatgatcct ccagcgcggg gatctcatgc tggagttctt cgcccacccc aacttgttta 4140
ttgcagctta taatggttac aaataaagca atagcatcac aaatttcaca aataaagcat 4200
ttttttcact gcattctagt tgtggtttgt ccaaactcat caatgtatct tatcatgtct 4260
gtataccgtc gacctctagc tagagcttgg cgtaatcatg gtcatagctg tttcctgtgt 4320
gaaattgtta tccgctcaca attccacaca acatacgagc cggaagcata aagtgtaaag 4380
cctggggtgc ctaatgagtg agctaactca cattaattgc gttgcgctca ctgcccgctt 4440
tccagtcggg aaacctgtcg tgccagctgc attaatgaat cggccaacgc gcggggagag 4500
gcggtttgcg tattgggcgc tcttccgctt cctcgctcac tgactcgctg cgctcggtcg 4560
ttcggctgcg gcgagcggta tcagctcact caaaggcggt aatacggtta tccacagaat 4620
caggggataa cgcaggaaag aacatgtgag caaaaggcca gcaaaaggcc aggaaccgta 4680
aaaaggccgc gttgctggcg tttttccata ggctccgccc ccctgacgag catcacaaaa 4740
atcgacgctc aagtcagagg tggcgaaacc cgacaggact ataaagatac caggcgtttc 4800
cccctggaag ctccctcgtg cgctctcctg ttccgaccct gccgcttacc ggatacctgt 4860
ccgcctttct cccttcggga agcgtggcgc tttctcatag ctcacgctgt aggtatctca 4920
Seite 3

C62387.ST25.txt
gttcggtgta ggtcgttcgc tccaagctgg gctgtgtgca cgaacccccc gttcagcccg 4980
accgctgcgc cttatccggt aactatcgtc ttgagtccaa cccggtaaga cacgacttat 5040
cgccactggc agcagccact ggtaacagga ttagcagagc gaggtatgta ggcggtgcta 5100
cagagttctt gaagtggtgg cctaactacg gctacactag aagaacagta tttggtatct 5160
gcgctctgct gaagccagtt accttcggaa aaagagttgg tagctcttga tccggcaaac 5220
aaaccaccgc tggtagcggt ggtttttttg tttgcaagca gcagattacg cgcagaaaaa 5280
aaggatctca agaagatcct ttgatctttt ctacggggtc tgacgctcag tggaacgaaa 5340
actcacgtta agggattttg gtcatgagat tatcaaaaag gatcttcacc tagatccttt 5400
taaattaaaa atgaagtttt aaatcaatct aaagtatata tgagtaaact tggtctgaca 5460
gttaccaatg cttaatcagt gaggcaccta tctcagcgat ctgtctattt cgttcatcca 5520
tagttgcctg actccccgtc gtgtagataa ctacgatacg ggagggctta ccatctggcc 5580
ccagtgctgc aatgataccg cgagacccac gctcaccggc tccagattta tcagcaataa 5640
accagccagc cggaagggcc gagcgcagaa gtggtcctgc aactttatcc gcctccatcc 5700
agtctattaa ttgttgccgg gaagctagag taagtagttc gccagttaat agtttgcgca 5760
acgttgttgc cattgctaca ggcatcgtgg tgtcacgctc gtcgtttggt atggcttcat 5820
tcagctccgg ttcccaacga tcaaggcgag ttacatgatc ccccatgttg tgcaaaaaag 5880
cggttagctc cttcggtcct ccgatcgttg tcagaagtaa gttggccgca gtgttatcac 5940
tcatggttat ggcagcactg cataattctc ttactgtcat gccatccgta agatgctttt 6000
ctgtgactgg tgagtactca accaagtcat tctgagaata gtgtatgcgg cgaccgagtt 6060
gctcttgccc ggcgtcaata cgggataata ccgcgccaca tagcagaact ttaaaagtgc 6120
tcatcattgg aaaacgttct tcggggcgaa aactctcaag gatcttaccg ctgttgagat 6180
ccagttcgat gtaacccact cgtgcaccca actgatcttc agcatctttt actttcacca 6240
gcgtttctgg gtgagcaaaa acaggaaggc aaaatgccgc aaaaaaggga ataagggcga 6300
cacggaaatg ttgaatactc atactcttcc tttttcaata ttattgaagc atttatcagg 6360
gttattgtct catgagcgga tacatatttg aatgtattta gaaaaataaa caaatagggg 6420
ttccgcgcac atttccccga aaagtgccac ctgacgtc 6458

<210> 2

<211> 7464

<212> DNA

<213> Artificial sequence


<220>

<223> Plasmid pMG10Ala7

<400> 2
Seite 4

C62387.ST25.txt
gacggatcgg gagatctccc gatcccctat ggtgcactct cagtacaatc tgctctgatg 60
ccgcatagtt aagccagtat ctgctccctg cttgtgtgtt ggaggtcgct gagtagtgcg 120
cgagcaaaat ttaagctaca acaaggcaag gcttgaccga caattgcatg aagaatctgc 180
ttagggttag gcgttttgcg ctgcttcgta agctgcaata aacaatcatt attttcattg 240
gatctgtgtg ttggtttttt gtgtgggctt gggggagggg gaggccagaa tgactccaag 300
agctacagga aggcaggtca gagaccccac tggacaaaca gtggctggac tctgcaccat 360
aacacacaat caacagggga gtgagctgga tcgagctaga gtccgttaca taacttacgg 420
taaatggccc gcctggctga ccgcccaacg acccccgccc attgacgtca ataatgacgt 480
atgttcccat agtaacgcca atagggactt tccattgacg tcaatgggtg gagtatttac 540
ggtaaactgc ccacttggca gtacatcaag tgtatcatat gccaagtacg ccccctattg 600
acgtcaatga cggtaaatgg cccgcctggc attatgccca gtacatgacc ttatgggact 660
ttcctacttg gcagtacatc tacgtattag tcatcgctat taccatggtg atgcggtttt 720
ggcagtacat caatgggcgt ggatagcggt ttgactcacg gggatttcca agtctccacc 780
ccattgacgt caatgggagt ttgttttggc accaaaatca acgggacttt ccaaaatgtc 840
gtaacaactc cgccccattg acgcaaatgg gcggtaggcg tgtacggtgg gaggtctata 900
taagcagagc tcgtttagtg aaccgtcaga tcgcctggag acgccatcca cgctgttttg 960
acctccatag aagacaccgg gaccgatcca gcctccgcgg ccgggaacgg tgcattggaa 1020
cgcggattcc ccgtgccaag agtgacgtaa gtaccgccta tagagtctat aggcccaccc 1080
ccttggcttc ttatgcatgc tatactgttt ttggcttggg gtctatacac ccccgcttcc 1140
tcatgttata ggtgatggta tagcttagcc tataggtgtg ggttattgac cattattgac 1200
cactccccta ttggtgacga tactttccat tactaatcca taacatggct ctttgccaca 1260
actctcttta ttggctatat gccaatacac tgtccttcag agactgacac ggactctgta 1320
tttttacagg atggggtctc atttattatt tacaaattca catatacaac accaccgtcc 1380
ccagtgcccg cagtttttat taaacataac gtgggatctc cacgcgaatc tcgggtacgt 1440
gttccggaca tgggctcttc tccggtagcg gcggagcttc tacatccgag ccctgctccc 1500
atgcctccag cgactcatgg tcgctcggca gctccttgct cctaacagtg gaggccagac 1560
ttaggcacag cacgatgccc accaccacca gtgtgccgca caaggccgtg gcggtagggt 1620
atgtgtctga aaatgagctc ggggagcggg cttgcaccgc tgacgcattt ggaagactta 1680
aggcagcggc agaagaagat gcaggcagct gagttgttgt gttctgataa gagtcagagg 1740
taactcccgt tgcggtgctg ttaacggtgg agggcagtgt agtctgagca gtactcgttg 1800
ctgccgcgcg cgccaccaga cataatagct gacagactaa cagactgttc ctttccatgg 1860
gtcttttctg cagtcacccg ggggatcctt cgaacgtagc tctagccacc atgcccatgg 1920
ggtctctgca accgctggcc accttgtacc tgctggggat gctggtcgct tcctgcctcg 1980
gaaactgggt gaatgtaata agtgatttga aaaaaattga agatcttatt caatctatgc 2040
Seite 5

C62387.ST25.txt
atattgatgc tactttatat acggaaagtg atgttcaccc cagttgcaaa gtaacagcaa 2100
tgaagtgctt tctcttggag ttacaagtta tttcacttga gtccggagat gcaagtattc 2160
atgatacagt agaaaatctg atcatcctag caaacaacag tttgtcttct aatgggaatg 2220
taacagaatc tggatgcaaa gaatgtgagg aactggagga aaaaaatatt aaagaatttt 2280
tggacagttt tgtacatatt gtcgacatgt tcatcaacac ttcggatccc aaatctgctg 2340
acaaaactca cacatgccca ccgtgcccag cacctgaact cctgggggga ccgtcagtct 2400
tcctcttccc cccaaaaccc aaggacaccc tcatgatctc ccggacccct gaggtcacgt 2460
gcgtggtggt ggacgtgagc cacgaagacc ctgaggtcaa gttcaactgg tacgtggacg 2520
gcgtggaggt gcataatgcc aagacaaagc cgcgggagga gcagtacaac agcacgtacc 2580
gtgtggtcag cgtcctcacc gtcctgcacc aggactggct gaatggcaag gagtacaagt 2640
gcaaggtctc caacaaagcc ctcccagccc ccatcgagaa aaccatctcc aaagccaaag 2700
ggcagccccg agaaccacag gtgtacaccc tgcccccatc ccgggatgag ctgaccaaga 2760
accaggtcag cctgacctgc ctggtcaaag gcttctatcc cagcgacatc gccgtggagt 2820
gggagagcaa tgggcagccg gagaacaact acaagaccac gcctcccgtg ctggactccg 2880
acggctcctt cttcctctac agcaagctca ccgtggacaa gagcaggtgg cagcagggga 2940
acgtcttctc atgctccgtg atgcatgagg ctctgcacaa ccactacacg cagaagagcc 3000
tctccctgtc tccgggtaaa tgatctagag ggcccgttta aacccgctga tcagcctcga 3060
ctgtgccttc tagttgccag ccatctgttg tttgcccctc ccccgtgcct tccttgaccc 3120
tggaaggtgc cactcccact gtcctttcct aataaaatga ggaaattgca tcgcattgtc 3180
tgagtaggtg tcattctatt ctggggggtg gggtggggca ggacagcaag ggggaggatt 3240
gggaagacaa tagcaggcat gctggggatg cggtgggctc tatggcttct gaggcggaaa 3300
gaaccagctg gggctctagg gggtatcccc acgcgccctg tagcggcgca ttaagcgcgg 3360
cgggtgtggt ggttacgcgc agcgtgaccg ctacacttgc cagcgcccta gcgcccgctc 3420
ctttcgcttt cttcccttcc tttctcgcca cgttcgccgg ctttccccgt caagctctaa 3480
atcgggggct ccctttaggg ttccgattta gtgctttacg gcacctcgac cccaaaaaac 3540
ttgattaggg tgatggttca cgtagtgggc catcgccctg atagacggtt tttcgccctt 3600
tgacgttgga gtccacgttc tttaatagtg gactcttgtt ccaaactgga acaacactca 3660
accctatctc ggtctattct tttgatttat aagggatttt gccgatttcg gcctattggt 3720
taaaaaatga gctgatttaa caaaaattta acgcgaatta attctgtgga atgtgtgtca 3780
gttagggtgt ggaaagtccc caggctcccc agcaggcaga agtatgcaaa gcatgcatct 3840
caattagtca gcaaccaggt gtggaaagtc cccaggctcc ccagcaggca gaagtatgca 3900
aagcatgcat ctcaattagt cagcaaccat agtcccgccc ctaactccgc ccatcccgcc 3960
cctaactccg cccagttccg cccattctcc gccccatggc tgactaattt tttttattta 4020
tgcagaggcc gaggccgcct ctgcctctga gctattccag aagtagtgag gaggcttttt 4080
Seite 6

C62387.ST25.txt
tggaggccta ggcttttgca aaaagctccc gggagcttgt atatccattt tcggatctga 4140
tcaagagaca ggatgaggat cgtttcgcat gattgaacaa gatggattgc acgcaggttc 4200
tccggccgct tgggtggaga ggctattcgg ctatgactgg gcacaacaga caatcggctg 4260
ctctgatgcc gccgtgttcc ggctgtcagc gcaggggcgc ccggttcttt ttgtcaagac 4320
cgacctgtcc ggtgccctga atgaactgca ggacgaggca gcgcggctat cgtggctggc 4380
cacgacgggc gttccttgcg cagctgtgct cgacgttgtc actgaagcgg gaagggactg 4440
gctgctattg ggcgaagtgc cggggcagga tctcctgtca tctcaccttg ctcctgccga 4500
gaaagtatcc atcatggctg atgcaatgcg gcggctgcat acgcttgatc cggctacctg 4560
cccattcgac caccaagcga aacatcgcat cgagcgagca cgtactcgga tggaagccgg 4620
tcttgtcgat caggatgatc tggacgaaga gcatcagggg ctcgcgccag ccgaactgtt 4680
cgccaggctc aaggcgcgca tgcccgacgg cgaggatctc gtcgtgaccc atggcgatgc 4740
ctgcttgccg aatatcatgg tggaaaatgg ccgcttttct ggattcatcg actgtggccg 4800
gctgggtgtg gcggaccgct atcaggacat agcgttggct acccgtgata ttgctgaaga 4860
gcttggcggc gaatgggctg accgcttcct cgtgctttac ggtatcgccg ctcccgattc 4920
gcagcgcatc gccttctatc gccttcttga cgagttcttc tgagcgggac tctggggttc 4980
gaaatgaccg accaagcgac gcccaacctg ccatcacgag atttcgattc caccgccgcc 5040
ttctatgaaa ggttgggctt cggaatcgtt ttccgggacg ccggctggat gatcctccag 5100
cgcggggatc tcatgctgga gttcttcgcc caccccaact tgtttattgc agcttataat 5160
ggttacaaat aaagcaatag catcacaaat ttcacaaata aagcattttt ttcactgcat 5220
tctagttgtg gtttgtccaa actcatcaat gtatcttatc atgtctgtat accgtcgacc 5280
tctagctaga gcttggcgta atcatggtca tagctgtttc ctgtgtgaaa ttgttatccg 5340
ctcacaattc cacacaacat acgagccgga agcataaagt gtaaagcctg gggtgcctaa 5400
tgagtgagct aactcacatt aattgcgttg cgctcactgc ccgctttcca gtcgggaaac 5460
ctgtcgtgcc agctgcatta atgaatcggc caacgcgcgg ggagaggcgg tttgcgtatt 5520
gggcgctctt ccgcttcctc gctcactgac tcgctgcgct cggtcgttcg gctgcggcga 5580
gcggtatcag ctcactcaaa ggcggtaata cggttatcca cagaatcagg ggataacgca 5640
ggaaagaaca tgtgagcaaa aggccagcaa aaggccagga accgtaaaaa ggccgcgttg 5700
ctggcgtttt tccataggct ccgcccccct gacgagcatc acaaaaatcg acgctcaagt 5760
cagaggtggc gaaacccgac aggactataa agataccagg cgtttccccc tggaagctcc 5820
ctcgtgcgct ctcctgttcc gaccctgccg cttaccggat acctgtccgc ctttctccct 5880
tcgggaagcg tggcgctttc tcatagctca cgctgtaggt atctcagttc ggtgtaggtc 5940
gttcgctcca agctgggctg tgtgcacgaa ccccccgttc agcccgaccg ctgcgcctta 6000
tccggtaact atcgtcttga gtccaacccg gtaagacacg acttatcgcc actggcagca 6060
gccactggta acaggattag cagagcgagg tatgtaggcg gtgctacaga gttcttgaag 6120
Seite 7

C62387.ST25.txt
tggtggccta actacggcta cactagaaga acagtatttg gtatctgcgc tctgctgaag 6180

ccagttacct tcggaaaaag agttggtagc tcttgatccg gcaaacaaac caccgctggt 6240

agcggtggtt tttttgtttg caagcagcag attacgcgca gaaaaaaagg atctcaagaa 6300

gatcctttga tcttttctac ggggtctgac gctcagtgga acgaaaactc acgttaaggg 6360

attttggtca tgagattatc aaaaaggatc ttcacctaga tccttttaaa ttaaaaatga 6420

agttttaaat caatctaaag tatatatgag taaacttggt ctgacagtta ccaatgctta 6480

atcagtgagg cacctatctc agcgatctgt ctatttcgtt catccatagt tgcctgactc 6540

cccgtcgtgt agataactac gatacgggag ggcttaccat ctggccccag tgctgcaatg 6600

ataccgcgag acccacgctc accggctcca gatttatcag caataaacca gccagccgga 6660

agggccgagc gcagaagtgg tcctgcaact ttatccgcct ccatccagtc tattaattgt 6720

tgccgggaag ctagagtaag tagttcgcca gttaatagtt tgcgcaacgt tgttgccatt 6780

gctacaggca tcgtggtgtc acgctcgtcg tttggtatgg cttcattcag ctccggttcc 6840

caacgatcaa ggcgagttac atgatccccc atgttgtgca aaaaagcggt tagctccttc 6900

ggtcctccga tcgttgtcag aagtaagttg gccgcagtgt tatcactcat ggttatggca 6960

gcactgcata attctcttac tgtcatgcca tccgtaagat gcttttctgt gactggtgag 7020

tactcaacca agtcattctg agaatagtgt atgcggcgac cgagttgctc ttgcccggcg 7080

tcaatacggg ataataccgc gccacatagc agaactttaa aagtgctcat cattggaaaa 7140

cgttcttcgg ggcgaaaact ctcaaggatc ttaccgctgt tgagatccag ttcgatgtaa 7200

cccactcgtg cacccaactg atcttcagca tcttttactt tcaccagcgt ttctgggtga 7260

gcaaaaacag gaaggcaaaa tgccgcaaaa aagggaataa gggcgacacg gaaatgttga 7320

atactcatac tcttcctttt tcaatattat tgaagcattt atcagggtta ttgtctcatg 7380

agcggataca tatttgaatg tatttagaaa aataaacaaa taggggttcc gcgcacattt 7440

ccccgaaaag tgccacctga cgtc 7464

<210> 3

<211> 1113

<212> DNA

<213> Artificial sequence

<220>

<223> DNA für mutiertes IL-15/Fc mit CD5-Leader

<400> 3
atgcccatgg ggtctctgca accgctggcc accttgtacc tgctggggat gctggtcgct 60

tcctgcctcg gaaactgggt gaatgtaata agtgatttga aaaaaattga agatcttatt 120

caatctatgc atattgatgc tactttatat acggaaagtg atgttcaccc cagttgcaaa 180

Seite 8

C62387.ST25.txt
gtaacagcaa tgaagtgctt tctcttggag ttacaagtta tttcacttga gtccggagat 240

gcaagtattc atgatacagt agaaaatctg atcatcctag caaacaacag tttgtcttct 300

aatgggaatg taacagaatc tggatgcaaa gaatgtgagg aactggagga aaaaaatatt 360

aaagaatttt tggacagttt tgtacatatt gtcgacatgt tcatcaacac ttcggatccc 420

aaatctgctg acaaaactca cacatgccca ccgtgcccag cacctgaact cctgggggga 480

ccgtcagtct tcctcttccc cccaaaaccc aaggacaccc tcatgatctc ccggacccct 540

gaggtcacgt gcgtggtggt ggacgtgagc cacgaagacc ctgaggtcaa gttcaactgg 600

tacgtggacg gcgtggaggt gcataatgcc aagacaaagc cgcgggagga gcagtacaac 660

agcacgtacc gtgtggtcag cgtcctcacc gtcctgcacc aggactggct gaatggcaag 720

gagtacaagt gcaaggtctc caacaaagcc ctcccagccc ccatcgagaa aaccatctcc 780

aaagccaaag ggcagccccg agaaccacag gtgtacaccc tgcccccatc ccgggatgag 840

ctgaccaaga accaggtcag cctgacctgc ctggtcaaag gcttctatcc cagcgacatc 900

gccgtggagt gggagagcaa tgggcagccg gagaacaact acaagaccac gcctcccgtg 960

ctggactccg acggctcctt cttcctctac agcaagctca ccgtggacaa gagcaggtgg 1020

cagcagggga acgtcttctc atgctccgtg atgcatgagg ctctgcacaa ccactacacg 1080

cagaagagcc tctccctgtc tccgggtaaa tga 1113

<210> 4

<211> 370

<212> PRT

<213> Artificial sequence


<220>

<223> Aminosäuresequenz des humanen CRB-15 mit Leader CD5

<400> 4

Met Pro Met Gly Ser Leu Gln Pro Leu Ala Thr Leu Tyr Leu Leu Gly
1               5                   10                  15

Met Leu Val Ala Ser Cys Leu Gly Asn Trp Val Asn Val Ile Ser Asp
            20                  25                  30

Leu Lys Lys Ile Glu Asp Leu Ile Gln Ser Met His Ile Asp Ala Thr
        35                  40                  45

Leu Tyr Thr Glu Ser Asp Val His Pro Ser Cys Lys Val Thr Ala Met
    50                  55                  60

Lys Cys Phe Leu Leu Glu Leu Gln Val Ile Ser Leu Glu Ser Gly Asp
65                  70                  75                  80

Seite 9

C62387.ST25.txt

Ala Ser Ile His Asp Thr Val Glu Asn Leu Ile Ile Leu Ala Asn Asn
                85                  90                  95

Ser Leu Ser Ser Asn Gly Asn Val Thr Glu Ser Gly Cys Lys Glu Cys
            100                 105                 110

Glu Glu Leu Glu Glu Lys Asn Ile Lys Glu Phe Leu Asp Ser Phe Val
        115                 120                 125

His Ile Val Asp Met Phe Ile Asn Thr Ser Asp Pro Lys Ser Ala Asp
    130                 135                 140

Lys Thr His Thr Cys Pro Pro Cys Pro Ala Pro Glu Leu Leu Gly Gly
145                 150                 155                 160

Pro Ser Val Phe Leu Phe Pro Pro Lys Pro Lys Asp Thr Leu Met Ile
                165                 170                 175

Ser Arg Thr Pro Glu Val Thr Cys Val Val Val Asp Val Ser His Glu
            180                 185                 190

Asp Pro Glu Val Lys Phe Asn Trp Tyr Val Asp Gly Val Glu Val His
        195                 200                 205

Asn Ala Lys Thr Lys Pro Arg Glu Glu Gln Tyr Asn Ser Thr Tyr Arg
    210                 215                 220

Val Val Ser Val Leu Thr Val Leu His Gln Asp Trp Leu Asn Gly Lys
225                 230                 235                 240

Glu Tyr Lys Cys Lys Val Ser Asn Lys Ala Leu Pro Ala Pro Ile Glu
                245                 250                 255

Lys Thr Ile Ser Lys Ala Lys Gly Gln Pro Arg Glu Pro Gln Val Tyr
            260                 265                 270

Thr Leu Pro Pro Ser Arg Asp Glu Leu Thr Lys Asn Gln Val Ser Leu
        275                 280                 285

Thr Cys Leu Val Lys Gly Phe Tyr Pro Ser Asp Ile Ala Val Glu Trp
    290                 295                 300

Glu Ser Asn Gly Gln Pro Glu Asn Asn Tyr Lys Thr Thr Pro Pro Val
305                 310                 315                 320

Leu Asp Ser Asp Gly Ser Phe Phe Leu Tyr Ser Lys Leu Thr Val Asp
                325                 330                 335

Lys Ser Arg Trp Gln Gln Gly Asn Val Phe Ser Cys Ser Val Met His
            340                 345                 350

Seite 10

C62387.ST25.txt

Glu Ala Leu His Asn His Tyr Thr Gln Lys Ser Leu Ser Leu Ser Pro
        355                 360                 365

Gly Lys
    370

<210>  5

<211>  371

<212>  PRT

<213>  Artificial sequence

<220>

<223>  Aminosäuresequenz des murinen IL-15/Fc (humanes mutiertes IL-15,
       muriner IgG2A) mit CD5-Leader

<400>  5

Met Pro Met Gly Ser Leu Gln Pro Leu Ala Thr Leu Tyr Leu Leu Gly
1               5                   10                  15

Met Leu Val Ala Ser Cys Leu Gly Asn Trp Val Asn Val Ile Ser Asp
            20                  25                  30

Leu Lys Lys Ile Glu Asp Leu Ile Gln Ser Met His Ile Asp Ala Thr
        35                  40                  45

Leu Tyr Thr Glu Ser Asp Val His Pro Ser Cys Lys Val Thr Ala Met
    50                  55                  60

Lys Cys Phe Leu Leu Glu Leu Gln Val Ile Ser Leu Glu Ser Gly Asp
65                  70                  75                  80

Ala Ser Ile His Asp Thr Val Glu Asn Leu Ile Ile Leu Ala Asn Asn
                85                  90                  95

Ser Leu Ser Ser Asn Gly Asn Val Thr Glu Ser Gly Cys Lys Glu Cys
            100                 105                 110

Glu Glu Leu Glu Glu Lys Asn Ile Lys Glu Phe Leu Asp Ser Phe Val
        115                 120                 125

His Ile Val Asp Met Phe Ile Asn Thr Ser Asp Pro Arg Gly Pro Thr
    130                 135                 140

Ile Lys Pro Cys Pro Pro Cys Lys Cys Pro Ala Pro Asn Leu Leu Gly
145                 150                 155                 160

Gly Pro Ser Val Phe Ile Phe Pro Pro Lys Ile Lys Asp Val Leu Met
                165                 170                 175

Seite 11

C62387.ST25.txt

Ile Ser Leu Ser Pro Ile Val Thr Cys Val Val Val Asp Val Ser Glu
            180                 185                 190

Asp Asp Pro Asp Val Gln Ile Ser Trp Phe Val Asn Asn Val Glu Val
        195                 200                 205

His Thr Ala Gln Thr Gln Thr His Arg Glu Asp Tyr Asn Ser Thr Leu
    210                 215                 220

Arg Val Val Ser Ala Leu Pro Ile Gln His Gln Asp Trp Met Ser Gly
225                 230                 235                 240

Lys Glu Phe Lys Cys Lys Val Asn Asn Lys Asp Leu Pro Ala Pro Ile
                245                 250                 255

Glu Arg Thr Ile Ser Lys Pro Lys Gly Ser Val Arg Ala Pro Gln Val
            260                 265                 270

Tyr Val Leu Pro Pro Pro Glu Glu Glu Met Thr Lys Lys Gln Val Thr
        275                 280                 285

Leu Thr Cys Met Val Thr Asp Phe Met Pro Glu Asp Ile Tyr Val Glu
    290                 295                 300

Trp Thr Asn Asn Gly Lys Thr Glu Leu Asn Tyr Lys Asn Thr Glu Pro
305                 310                 315                 320

Val Leu Asp Ser Asp Gly Ser Tyr Phe Met Tyr Ser Lys Leu Arg Val
                325                 330                 335

Glu Lys Lys Asn Trp Val Glu Arg Asn Ser Tyr Ser Cys Ser Val Val
            340                 345                 350

His Glu Gly Leu His Asn His His Thr Thr Lys Ser Phe Ser Arg Thr
        355                 360                 365

Pro Gly Lys
    370

Seite 12

## Patentansprüche

1. Expressionssystem enthaltend eine oder mehrere Nukleinsäure(n) umfassend
a) mindestens eine Nukleinsäure für ein IL-15/Fc-Fusionsprotein,
b) mindestens einen Promotor und
c) mindestens eine Nukleinsäure für einen CD5-Leader,
wobei der Promotor und die Nukleinsäure für den CD5-Leader funktionsfähig mit der Nukleinsäure für das IL-15/Fc-Fusionsprotein verknüpft sind.

2. Expressionssystem nach Anspruch 1, wobei der Promotor ein CMV-Promotor ist.

3. Expressionssystem nach Anspruch 1 oder 2, wobei der Promotor Bestandteil einer transkriptionsregulierenden Einheit ist, die zusätzlich ein Intron, insbesondere Intron A, enthält.

4. Expressionssystem nach einem der Ansprüche 1 bis 3, wobei es sich bei dem Fc-Teil des Fusionsproteins um ein Fc-Fragment eines Immunglobulins G handelt.

5. Expressionssystem nach einem der Ansprüche 1 bis 4 enthaltend zusätzlich
d) mindestens eine Nukleinsäure für ein selektionierbares Markergen.

6. Expressionssystem nach einem der Ansprüche 1 bis 5 enthaltend zusätzlich mindestens eine Nukleinsäure für ein Polyadenylierungssignal.

7. Expressionssystem nach einem der Ansprüche 1 bis 6 enthaltend zusätzlich Ribosomen, Aminosäuren und/oder t-RNAs.

8. Expressionssystem nach einem der Ansprüche 1 bis 7, wobei das Expressionssystem nur eine Nukleinsäure umfasst.

9. Expressionssystem nach einem der Ansprüche 1 bis 8 enthaltend eine Nukleinsäure mit der Sequenz von SEQ ID Nr. 1, SEQ ID Nr. 2 oder SEQ ID Nr. 3 oder eine Nukleinsäure, die für ein Polypeptid der SEQ ID NR. 4 oder SEQ ID NR. 5 kodiert.

10. Nukleinsäure enthaltend die Bestandteile a) bis c) der Ansprüche 1 bis 4 und optional Bestandteil d) aus Anspruch 5.

11. Nukleinsäure enthaltend die Sequenz von SEQ ID Nr. 1, SEQ ID Nr. 2 oder 3 oder eine Nukleinsäure, die für ein Polypeptid der SEQ ID NR. 4 oder SEQ ID NR. 5 kodiert.

12. Wirtszelle enthaltend ein Expressionssystem gemäß einem der Ansprüche 1 bis 9 oder eine Nukleinsäure gemäß Anspruch 10 oder 11.

13. Wirtszelle nach Anspruch 12, wobei es sich um eine Säugerzelle handelt.

14. Wirtszelle nach Anspruch 12 oder 13, wobei es sich um eine Zelle der Zelllinie CHO oder deren Derivate, insbesondere eine CHO-K1-Zelllinie, handelt.

15. Verfahren zur Herstellung eines IL-15/Fc-Fusionsproteins umfassend
a. Bereitstellen einer Wirtszelle nach einem der Ansprüche 12 bis 14,
b. Kultivieren der Wirtszelle,
c. gegebenenfalls Selektionieren und
d. Isolieren des exprimierten IL-15/Fc-Fusionsproteins.

16. Verfahren nach Anspruch 15, wobei es sich bei der Wirtzelle um eine Säugerzelle, vorzugsweise um eine Zelle der Zelllinie CHO oder deren Derivate, besonders bevorzugt um eine CHO-K1-Zelllinie, handelt.

17. Verfahren nach Anspruch 15 oder 16, wobei das IL-15/Fc-Fusionsprotein in einer Menge von mindestens 10 pg/(Zelle x Tag), vorzugsweise in einer Menge mindestens 15 pg/(Zelle x Tag), hergestellt wird.

18. Verwendung eines Expressionssystems nach einem der Ansprüche 1 bis 9, einer Nukleinsäure nach Anspruch 10 oder 11 oder einer Wirtzelle nach einem der Ansprüche 12 bis 14 zur Herstellung eines IL-15-Fc-Fusionsproteins.

19. Verwendung eines CD5-Leaders zur Expression eines Proteins in CHO-Zellen und ihren Derivaten, insbesondere CHO-K1-Zellen.

20. Verwendung nach Anspruch 19, wobei die Expression des Proteins von einem CMV-Promotor, insbesondere in Kombination mit Intron A, reguliert wird.
